(19) Europäisches Patentamt
European Patent Office
Office européen des brevets

(11) Veröffentlichungsnummer: **0 234 250**
**A2**

(12) # EUROPÄISCHE PATENTANMELDUNG

(21) Anmeldenummer: 87100640.9

(22) Anmeldetag: 19.01.87

(51) Int. Cl.³: C 07 D 239/42
C 07 D 251/16, C 07 D 251/46
C 07 D 239/52, C 07 D 213/60
C 07 D 237/10, C 07 D 253/04
C 07 D 253/06, C 07 D 257/00
C 07 D 521/00, A 01 N 47/08

(30) Priorität: 30.01.86 DE 3602679

(43) Veröffentlichungstag der Anmeldung:
02.09.87 Patentblatt 87/36

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kirsten, Rolf, Dr.
Carl-Langhans-Strasse 27
D-4019 Monheim(DE)

(72) Erfinder: Kluth, Joachim, Dr.
Kurt-Schumacher Strasse 9
D-4018 Langenfeld(DE)

(72) Erfinder: Müller, Klaus-Helmut, Dr.
Bockhackstrasse 53
D-4000 Düsseldorf 13(DE)

(72) Erfinder: Pfister, Theodor, Dr.
Lichtenberger Strasse 30
D-4019 Monheim(DE)

(72) Erfinder: Riebel, Hans-Jochem Dr.
In der Beek 92
D-5600 Wuppertal 1(DE)

(72) Erfinder: Santel, Hans-Joachim Dr.
Grünstrasse 9a
D-5090 Leverkusen 1(DE)

(72) Erfinder: Schmidt, Robert R., Dr.
Im Waldwinkel 110
D-5060 Bergisch Gladbach 2(DE)

(54) **Acylierte Sulfonylguanidine.**

(57) Die Erfindung betrifft neue acylierte Sulfonylguanidine der allgemeinen Formel (I)

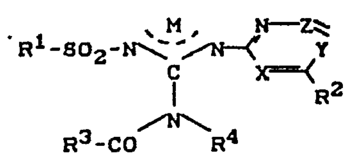

(I)

(worin die Reste X, Y, Z $R^1$, $R^2$, $R^3$, $R^4$ und M die in der Beschreibung angegebenen Bedeutungen haben), sowie Addukte von Verbindungen der Formel (I) mit starken Säuren, Verfahren und neue Zwischenprodukte zu ihrer Herstellung und ihre Verwendung als Herbizide.

EP 0 234 250 A2

BAYER AKTIENGESELLSCHAFT 5090 Leverkusen, Bayerwerk
Konzernverwaltung RP
Patentabteilung Bi/mö-c
Ib

# Acylierte Sulfonylguanidine

Die Erfindung betrifft neue acylierte Sulfonylguanidine, Verfahren und neue Zwischenprodukte zu ihrer Herstellung und ihre Verwendung als Herbizide.

Es ist bekannt, daß bestimmte Guanidine, wie z. B. N'-(4,6-Dimethyl-pyrimidin-2-yl)-N''-hydroxy-N'''-(2-chlorbenzolsulfonyl)-guanidin, herbizid wirksam sind. Die Wirkung dieser Verbindungen ist jedoch nicht immer ganz befriedigend (vergl. EP-OS 117 014).

Es wurden nun neue acylierte Sulfonylguanidine der allgemeinen Formel (I)

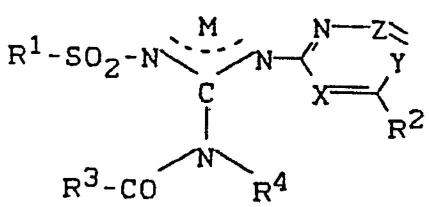

(I)

0234250

in welcher

$R^1$ für einen gegebenenfalls substituierten Rest aus der Reihe Alkyl, Aralkyl und Aryl mit jeweils bis zu 10 Kohlenstoffatomen oder für einen gegebenenfalls substituierten und/oder gegebenenfalls anellierten fünf- oder sechsgliedrigen aromatischen Heterocyclus, welcher ein Sauerstoff- oder ein Schwefelstoffatom und/ oder 1 bis 2 Stickstoffatome enthält, steht,

$R^2$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine -$CR^5$-Gruppierung steht, worin

$R^5$ für Wasserstoff, Halogen, Cyano, $C_1$-$C_4$-Alkyl, Formyl, $C_1$-$C_4$-Alkyl-carbonyl oder $C_1$-$C_4$-Alkoxy-carbonyl steht,

Z für Stickstoff oder eine -$CR^6$-Gruppierung steht, worin

$R^6$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

in welcher weiter

$R^3$ für einen Rest $R^7$ oder für einen Rest $-NHR^8$ steht, wobei

$R^7$ für einen gegebenenfalls substituierten Rest aus der Reihe Alkyl, Alkenyl, Alkinyl, Alkoxy, Alkenyloxy, Alkinyloxy, Cycloalkyl, Dialkylamino, Aralkyl, Aryl, Aralkoxy, Aryloxy, Alkylthio, Aralkylthio und Arylthio mit jeweils bis zu 10 Kohlenstoffatomen steht und

$R^8$ für einen gegebenenfalls substituierten Rest aus der Reihe Alkyl, Cycloalkyl, Aralkyl, Aryl oder Arylsulfonyl mit jeweils bis zu 10 Kohlenstoffatomen steht,

in welcher weiter

$R^4$ für Wasserstoff oder für einen gegebenenfalls substituierten Rest aus der Reihe Alkyl, Alkenyl, Alkinyl, Cycloalkyl, Aralkyl und Aryl mit jeweils bis zu 10 Kohlenstoffatomen steht oder

$R^4$ für den Rest $-OR^9$ steht, worin

$R^9$ für Wasserstoff oder einen gegebenenfalls substituierten Rest aus der Reihe Alkyl, Alkenyl, Alkinyl, Cycloalkyl, Cycloalkylalkyl, Aralkyl und Aryl mit jeweils bis zu 10 Kohlenstoffatomen steht,

in welcher weiter

$R^4$ für den Rest $-NR^{10}R^{11}$ steht, worin

$R^{10}$ für Wasserstoff oder gegebenenfalls substituiertes $C_1$-$C_4$-Alkyl steht und

$R^{11}$ für Wasserstoff oder einen gegebenenfalls substituierten Rest aus der Reihe Alkyl, Alkenyl, Alkinyl, Cycloalkyl, Aralkyl, Aryl, Alkylcarbonyl, Alkoxycarbonyl, Alkylsulfonyl, Arylsulfonyl mit jeweils bis zu 10 Kohlenstoffatomen oder für einen gegebenenfalls substituierten fünf- oder sechsgliedriegen aromatischen Heterocyclus, welcher ein Sauerstoff- oder ein Schwefelatom und/oder 1 bis 3 Stickstoffatome enthält, steht und

M für Wasserstoff, ein Metalläquivalent oder für den Rest $-COR^7$ steht, worin

$R^7$ die oben angegebenen Bedeutungen hat,

sowie Addukte von Verbindungen der Formel (I) mit starken Säuren gefunden.

Die allgemeine Formel (I) steht - wenn M für Wasserstoff steht - für die einzelnen Tautomeren der Formeln (IA) und (IB)

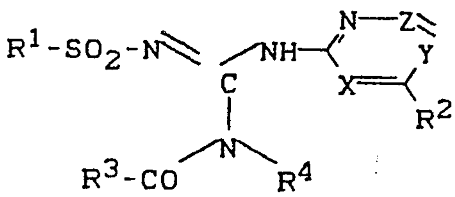

(IA)

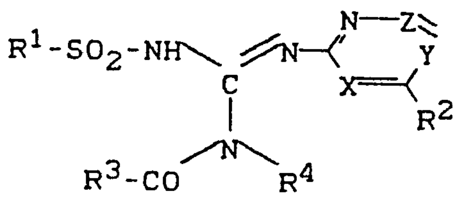

(IB)

in welchen

X, Y, Z, $R^1$, $R^2$, $R^3$ und $R^4$ die oben angegebenen Bedeutungen haben,

sowie für Gemische der Tautomeren (IA) und (IB).

Das Mischungsverhältnis hängt von aggregationsbestimmenden Faktoren wie z. B. Temperatur, Lösungsmittel und Konzentration ab.

Für den Fall, daß M für den Rest $-COR^7$ steht, sind die folgenden Isomeren der Formeln (IC) und (ID) möglich:

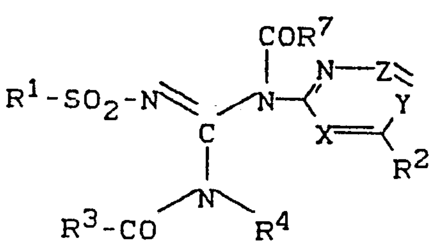

(IC)

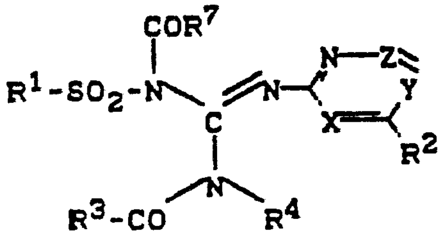 (ID)

in welchen

X, Y, Z, $R^1$, $R^2$, $R^3$, $R^4$ und $R^7$ die oben angegebenen Bedeutungen haben.

Man erhält die neuen acylierten Sulfonylguanidine der Formel (I)

(a) für den Fall, daß $R^3$ für den Rest $R^7$ steht, worin $R^7$ die oben angegebenen Bedeutungen hat, und M für Wasserstoff steht, wenn man Sulfonylguanidin-Derivate der Formel (II)

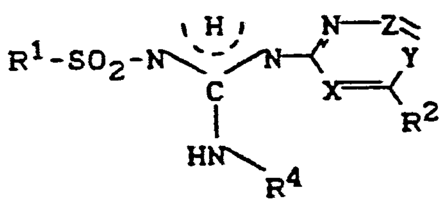 (II)

in welcher

X, Y, Z, $R^1$, $R^2$ und $R^4$ die oben angegebenen Bedeutungen haben,

mit Acylierungsmitteln der Formel (III)

$R^7COW$ (III)

in welcher

W für Halogen oder für den Rest $R^7COO$- steht und

$R^7$ die oben angegebenen Bedeutungen hat,

gegebenenfalls in Gegenwart von Säureakzeptoren und gegebenenfalls in Gegenwart von Verdünnungsmitteln umsetzt;

oder

(b) für den Fall, daß $R^3$ für den Rest $-NHR^8$ steht, worin $R^8$ die oben angegebenen Bedeutungen hat, und M für Wasserstoff steht, wenn man Sulfonylguanidin-Derivate der Formel (II)

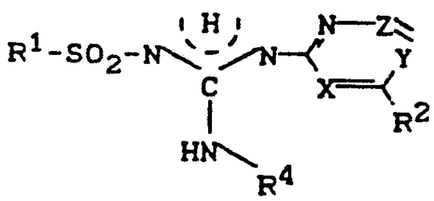

(II)

in welcher

X, Y, Z, $R^1$, $R^2$ und $R^4$ die oben angegebenen Bedeutungen haben,

mit Isocyanaten der Formel (IV)

$$R^8\text{-NCO} \qquad \text{(IV)}$$

in welcher

$R^8$ die oben angegebenen Bedeutungen hat,

gegebenenfalls in Gegenwart von Katalysatoren und gegebenenfalls in Gegenwart von Verdünnungsmitteln umsetzt;

oder

(c) für den Fall, daß M für Wasserstoff steht, wenn man Guanidin-Derivate der Formel (V)

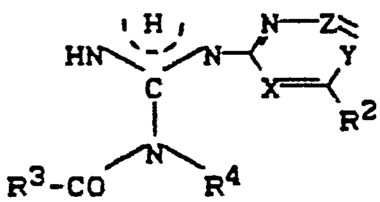

(V)

in welcher

X, Y, Z, $R^2$, $R^3$ und $R^4$ die oben angegebenen Bedeutungen haben,

mit Sulfonsäurechloriden der Formel (VI)

$$R^1\text{-}SO_2Cl \quad (VI)$$

in welcher

$R^1$ die oben angegebenen Bedeutungen hat,

in Gegenwart von Säureakzeptoren und gegebenenfalls in Gegenwart von Verdünnungsmitteln umsetzt;

oder

(d) für den Fall, daß M für ein Metalläquivalent steht, wenn man die nach den oben unter (a), (b) und (c) angegebenen Verfahren erhältlichen Verbindungen der Formel (I), in welcher M für Wasserstoff steht und X, Y, Z, $R^1$, $R^2$, $R^3$ und $R^4$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder

(e) für den Fall, daß 1:1-Addukte von Verbindungen der Formel (I) mit starken Säuren herzustellen sind,

wenn man Verbindungen der Formel (I), in welcher M für Wasserstoff steht und X, Y, Z, $R^1$, $R^2$, $R^3$ und $R^4$ die oben angegebenen Bedeutungen haben,

mit starken Säuren, gegebenenfalls in Gegenwart von Verdünnungsmitteln umsetzt;

oder

(f) für den Fall, daß M für den Rest $R^7CO$- steht, worin $R^7$ die oben angegebenen Bedeutungen hat,

(α) wenn man die nach den oben unter (a), (b) und (c) angegebenen Verfahren erhältlichen Verbindungen der Formel (I), in welcher M für Wasserstoff steht und X, Y, Z, $R^1$, $R^2$, $R^3$ und $R^4$ die oben angegebenen Bedeutungen haben,

mit Acylierungsmitteln der Formel (III)

$R^7COW$ (III)

in welcher

W und $R^7$ die oben angegebenen Bedeutungen haben,

gegebenenfalls in Gegenwart von Säureakzeptoren und gegebenenfalls in Gegenwart von Verdünnungsmitteln umsetzt,

oder

(β) wenn man Sulfonylguanidin-Derivate der Formel (II)

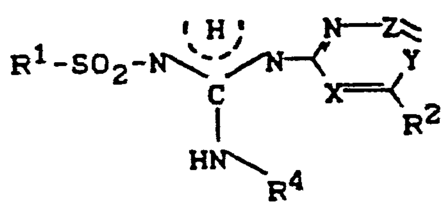 (II)

in welcher

X, Y, Z, $R^1$, $R^2$ und $R^4$ die oben angegebenen Bedeutungen haben,

mit mindestens der zweifachen molaren Menge Acylierungsmittel der Formel (III)

$R^7COW$ (III)

in welcher

W und $R^7$ die oben angegebenen Bedeutungen haben,

in Gegenwart von Säureakzeptoren und gegebenenfalls in Gegenwart von Verdünnungsmitteln umsetzt.

Die neuen acylierten Sulfonylguanidine der Formel (I) und ihre Addukte mit starken Säuren zeichnen sich durch starke herbizide Wirksamkeit aus.

Überraschenderweise zeigen die neuen Verbindungen der Formel (I) erheblich bessere herbizide Wirkung als vorbekannte Guanidine gleicher Wirkungsrichtung.

Gegenstand der Erfindung sind vorzugsweise Verbindungen der Formel (I), in welcher

$R^1$ für den Rest 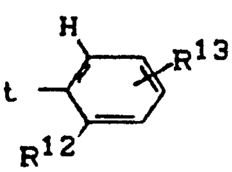 steht, worin

$R^{12}$ und $R^{13}$ gleich oder verschieden sind und für Wasserstoff, Halogen [wie insbesondere Fluor, Chlor, Brom und/oder Iod], Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-

Alkoxycarbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxycarbonyl substituiert ist], $C_3$-$C_6$-Alkinyloxy, $C_3$-$C_6$-Alkinylthio oder für den Rest $-S(O)_p-R^{14}$ stehen, wobei

p für die Zahlen 1 oder 2 steht und

$R^{14}$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxyamino, $C_1$-$C_4$-Alkoxy-$C_1$-$C_4$-alkylamino, $C_1$-$C_4$-Alkylamino oder Di-($C_1$-$C_4$-alkyl)-amino steht,

$R^{12}$ und $R^{13}$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest

$-CO-R^{15}$ stehen, wobei

$R^{15}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^{12}$ und $R^{13}$ weiterhin für $C_1$-$C_4$-Alkylsulfonyloxy, Di-($C_1$-$C_4$-alkyl)-aminosulfonylamino oder für den Rest $-CH=N-R^{16}$ stehen, wobei

$R^{16}$ für gegebenenfalls durch Fluor, Chlor, Cyano, Carboxy, $C_1$-$C_4$-Alkoxy, Carbonyl, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl,

für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkylsulfonylamino oder für gegebenenfalls durch Fluor, Chlor, Brom oder Methyl substituiertes Phenylsulfonylamino steht,

worin weiter

$R^1$ für den Rest 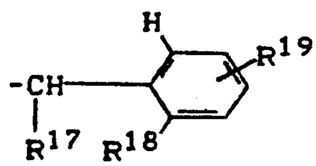 steht, worin

$R^{17}$ für Wasserstoff oder $C_1$-$C_4$-Alkyl steht,

$R^{18}$ und $R^{19}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest 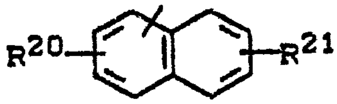 steht, worin

$R^{20}$ und $R^{21}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter

$R^1$ für den Rest 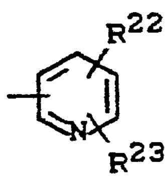 steht, worin

$R^{22}$ und $R^{23}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; worin weiter

$R^1$ für den Rest 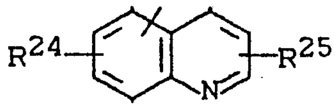 steht, worin

$R^{24}$ und $R^{25}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter

$R^1$ für den Rest 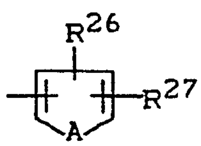 steht, worin

$R^{26}$ und $R^{27}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], Di-($C_1$-$C_4$-alkyl)-amino-sulfonyl oder $C_1$-$C_4$-Alkoxycarbonyl stehen, und

A für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter

$R^1$ für den Rest 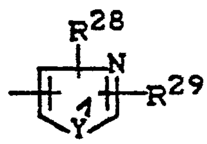 steht, worin

$R^{28}$ für Wasserstoff, $C_1$-$C_5$-Alkyl oder Halogen steht,

$R^{29}$ für Wasserstoff oder $C_1$-$C_5$-Alkyl steht und

$Y^1$ für Schwefel oder die Gruppierung N-$R^{30}$ steht, wobei

$R^{30}$ für Wasserstoff oder $C_1$-$C_5$-Alkyl steht,

in welcher weiter

$R^2$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine $-CR^5$-Gruppierung steht, worin

$R^5$ für Wasserstoff, Fluor, Chlor, Brom, Cyano, Methyl, Formyl, Acetyl, Methoxycarbonyl oder Ethoxycarbonyl steht,

und

Z für Stickstoff oder eine $-CR^6$-Gruppierung steht, worin

$R^6$ für Wasserstoff, Fluor, Chlor, Brom, Methyl, Ethyl, Methoxy, Ethoxy, Propoxy, Isopropoxy, Methylthio, Ethylthio, Methylamino, Ethylamino, Dimethylamino oder Diethylamino steht,

in welcher weiter

$R^3$ für den Rest $R^7$ oder für einen Rest $-NHR^8$ steht, wobei

$R^7$ für $C_1$-$C_8$-Alkyl, $C_1$-$C_8$-Alkoxy oder $C_1$-$C_8$-Alkylthio [welche gegebenenfalls durch Fluor, Chlor, Brom, Cyano, Nitro, $C_1$-$C_4$-Alkoxy-carbonyl, Di-

($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenyl oder $C_2$-$C_8$-Alkinyl [welche gegebenenfalls durch Fluor, Chlor, Brom, Cyano, Nitro, $C_1$-$C_4$-Alkoxy, $C_1$-$C_4$-Alkylthio oder Phenyl substituiert sind], für $C_2$-$C_8$-Alkenyloxy oder $C_2$-$C_8$-Alkinyloxy [welche gegebenenfalls durch Fluor, Chlor, Brom, Cyano, Nitro, $C_1$-$C_4$-Alkoxy, $C_1$-$C_4$-Alkylthio oder Phenyl substituiert sind], für $C_3$-$C_6$-Cycloalkyl oder Di-($C_1$-$C_4$-alkyl)-amino steht [welches gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für Phenoxy, Benzyloxy, für Phenylthio oder Benzylthio [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], sowie für Benzyl oder Phenyl steht {welche gegebenenfalls durch einen oder mehrere Reste aus der Reihe Halogen, Cyano, Nitro, $C_1$-$C_4$-Alkyl [welches gegebenenfalls durch Fluor, Chlor, Brom, Cyano, Carboxy, $C_1$-$C_4$-Alkoxy-carbonyl, Di-($C_1$-$C_4$-alkyl)-amino-carbonyl, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl oder Phenyl substituiert

ist], $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], $C_2$-$C_6$-Alkinyl [welches gegebenenfalls durch Fluor, Chlor, Brom, Cyano, $C_1$-$C_4$-Alkoxy-carbonyl oder Phenyl substituiert ist], $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkenylthio [welches gegebenenfalls durch Fluor, Chlor, Brom, Cyano oder $C_1$-$C_4$-Alkoxy-carbonyl substituiert ist], $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], Phenyl, Phenoxy, Di-($C_1$-$C_4$-alkyl)-amino-sulfonyl, $C_1$-$C_6$-Alkoxy-carbonyl, $C_3$-$C_6$-Cycloalkoxy-carbonyl oder Di-($C_1$-$C_4$-alkyl)-amino-carbonyl substituiert sind und/oder gegebenenfalls benzanneliert sind} und

$R^8$ für $C_1$-$C_6$-Alkyl, Cyclohexyl, Benzyl, Phenyl, Naphthyl, Phenylsulfonyl oder Naphthylsulfonyl steht (wobei die genannten aromatischen Reste gegebenenfalls durch Fluor, Chlor, Brom, $C_1$-$C_4$-Alkyl, $C_1$-$C_4$-Alkoxy, Trifluormethyl, $C_1$-$C_2$-Fluoralkoxy, $C_1$-$C_2$-Chlorfluoralkoxy, $C_1$-$C_2$-Chloralkoxy, $C_1$-$C_4$-Alkylthio, $C_1$-$C_4$-Alkylsul-

finyl, $C_1$-$C_4$-Alkylsulfonyl, Dimethylaminosulfonyl, N-Methoxy-N-methyl-aminosulfonyl und/oder $C_1$-$C_4$-Alkoxycarbonyl substituiert sind);

in welcher weiter

$R^4$ für Wasserstoff, $C_1$-$C_6$-Alkyl [welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht, oder

$R^4$ für den Rest $-OR^9$ steht, worin

$R^9$ für Wasserstoff, $C_1$-$C_8$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist], $C_3$-$C_6$-Alkenyl [welches gegebenenfalls durch Fluor oder Chlor substituiert ist], $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-

carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$- Alkoxy-carbonyl substituiert sind] steht und

in welcher weiter

$R^4$ für den Rest $-N\langle^{R^{10}}_{R^{11}}$ steht, worin

$R^{10}$ für Wasserstoff oder $C_1$-$C_4$-Alkyl steht und

$R^{11}$ für Wasserstoff, $C_1$-$C_4$-Alkyl [welches gegebenenfalls durch Halogen, Cyano, Nitro, $C_1$-$C_4$-Alkoxy oder $C_1$-$C_4$-Alkoxy-carbonyl substituiert ist], $C_3$-$C_6$-Cycloalkyl, Phenyl-$C_1$-$C_2$-alkyl, Phenyl [welche gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiert sind], für $C_1$-$C_4$-Alkyl-carbonyl, $C_1$-$C_4$-Alkoxy-carbonyl, $C_1$-$C_4$-Alkylsulfonyl, $C_1$-$C_4$-Halogenalkylsulfonyl oder Phenylsulfonyl [welches gegebenenfalls durch Halogen, Cyano, Nitro, $C_1$-$C_4$-Alkyl, $C_1$-$C_4$-Halogenalkyl, $C_1$-$C_4$-Alkoxy, $C_1$-$C_2$-Halogenalkoxy oder $C_1$-$C_4$-

Alkoxy-carbonyl substituiert ist] oder für ein Pyrimidinylrest steht und

M für Wasserstoff oder ein Natrium-, Kalium-, oder Calciumäquivalent oder für den Rest $-COR^7$ steht, worin $R^7$ die oben angegebenen bevorzugten Bedeutungen hat.

Gegenstand der Erfindung sind weiter vorzugsweise Addukte von Verbindungen der Formel (I) - wie vorausgehend definiert - mit Halogenwasserstoffsäuren, wie Hydrogenfluorid, Hydrogenchlorid, Hydrogenbromid, Hydrogeniodid, mit Schwefelsäure, mit gegebenenfalls durch Fluor und/oder Chlor substituierten Alkansulfonsäuren mit 1 bis 4 Kohlenstoffatomen oder auch mit Benzol- oder Naphthalinsulfonsäuren, welche gegebenenfalls durch Fluor, Chlor, Brom oder Methyl substituiert sind.

Gegenstand der Erfindung sind insbesondere Verbindungen der Formel (I), in welcher

(A) $R^1$ für den Rest 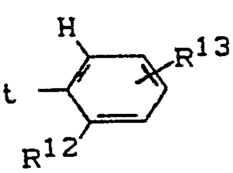 steht worin

$R^{12}$ für Fluor, Chlor, Brom, Methyl, Trifluormethyl, Methoxy, Difluormethoxy, Trifluormethoxy, $C_1$-$C_3$-Alkylthio, Difluormethylthio, Trifluormethylthio, $C_1$-$C_3$-Alkylsulfinyl, $C_1$-$C_3$-Alkylsulfonyl, Dimethylaminosulfonyl, Diethylaminosulfonyl, N-Methoxy-N-methyl-

aminosulfonyl, Phenyl, Phenoxy, $C_1$-$C_3$-Alkoxy-carbonyl, $C_1$-$C_3$-Alkoxyamino-sulfonyl, $C_1$-$C_3$-Alkoxyamino-carbonyl oder $C_1$-$C_3$-Alkylamino-carbonyl steht und

$R^{13}$ für Wasserstoff steht; worin weiter

$R^2$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine $-CR^5$-Gruppierung steht, worin

$R^5$ für Wasserstoff, Fluor, Chlor, Brom, Methyl, Formyl, Acetyl, Methoxycarbonyl oder Ethoxycarbonyl steht,

und

Z für Stickstoff oder eine $-CR^6$-Gruppierung steht, worin

$R^6$ für Wasserstoff, Fluor, Chlor, Brom, Methyl, Ethyl, Methoxy, Ethoxy, Propoxy, Isopropoxy, Methylthio, Ethylthio, Methylamino, Ethylamino, Dimethylamino oder Diethylamino steht,

in welcher weiter

$R^3$ für einen Rest $R^7$ steht, wobei

$R^7$ für $C_1$-$C_4$-Alkyl [welches gegebenenfalls durch Fluor und/oder Chlor substituiert ist], für Di-($C_1$-$C_2$-alkyl)-amino, für Benzyl oder Phenyl [welche gegebenenfalls durch Fluor, Chlor, Brom, Cyano, Nitro, $C_1$-$C_4$-Alkyl, Trifluormethyl, $C_1$-$C_4$-Alkoxy, Trifluormethoxy, Difluormethoxy, $C_1$-$C_4$-Alkylthio, $C_1$-$C_4$-Alkoxycarbonyl, $C_1$-$C_4$-Alkylsulfinyl, $C_1$-$C_4$-Alkylsulfonyl, Dimethylaminosulfonyl, Phenyl oder Phenoxy substituiert sind], steht

in welcher weiter

$R^4$ für den Rest -O-$R^9$ steht, worin

$R^9$ für $C_1$-$C_4$-Alkyl [welches gegebenenfalls durch Fluor oder Chlor substituiert ist], für $C_3$-$C_6$-Alkenyl [welches gegebenenfalls durch Chlor substituiert ist], für $C_1$-$C_3$-Alkoxy-carbonyl-$C_1$-$C_2$-alkyl, Phenyl, Phenylethyl oder Benzyl [welche gegebenenfalls durch Fluor, Chlor, Nitro, Methyl, Methoxy oder $C_1$-$C_2$-Alkoxy-carbonyl substituiert sind] steht;

in welcher weiter

$R^4$ für den Rest $-N\langle{}^{R^{10}}_{R^{11}}$ steht, worin

$R^{10}$ für Wasserstoff oder Methyl steht und

$R^{11}$ für Wasserstoff, $C_1$-$C_3$-Alkyl [welches gegebenenfalls durch Fluor, Chlor, Cyano, $C_1$-$C_2$-Alkoxy oder $C_1$-$C_2$-Alkoxy-carbonyl substituiert ist], für $C_3$-$C_6$-Cycloalkyl, Phenyl, Benzyl oder Phenylethyl [welche gegebenenfalls durch Fluor, Chlor, Brom, Nitro, Cyano, $C_1$-$C_2$-Alkyl, $C_1$-$C_2$-Alkoxy oder $C_1$-$C_2$-Alkoxy-carbonyl substituiert sind], für Acetyl, Methoxycarbonyl, Benzolsulfonyl oder Toluolsulfonyl steht;

in welcher weiter

M für Wasserstoff oder ein Natrium-, Kalium- oder Calciumäquivalent oder für den Rest $-COR^7$ steht, worin $R^7$ die oben angegebenen besonders bevorzugten Bedeutungen hat; und

(B) X, Y, Z, $R^1$, $R^2$, $R^4$ und M die oben unter (A) angegebenen Bedeutungen haben und

$R^3$ für einen Rest $-NHR^8$ steht, worin

$R^8$ für $C_1$-$C_4$-Alkyl, Cyclohexyl, Benzyl, Phenyl oder Phenylsulfonyl steht (wobei die Phenylreste gegebenenfalls durch Fluor, Chlor, $C_1$-$C_2$-Alkyl, $C_1$-$C_2$-Alkoxy, Trifluormethyl, Trifluormethoxy, Chlorfluormethoxy, Chlordifluormethoxy, Dichlormethoxy, 2-Chlorethoxy, $C_1$-$C_2$-Alkylthio, $C_1$-$C_2$-Alkylsulfinyl, $C_1$-$C_2$-Alkylsulfonyl, Dimethylaminosulfonyl, N-Methoxy-N-methylaminosulfonyl und/oder $C_1$-$C_2$-Alkoxycarbonyl substituiert sind).

Gegenstand der Erfindung sind weiter insbesondere Addukte von Verbindungen der Formel (I) - wie vorausgehend definiert - mit Halogenwasserstoffsäuren, wie Hydrogenchlorid, Hydrogenbromid und Hydrogeniodid, mit Schwefelsäure, mit gegebenenfalls durch Fluor und/oder Chlor substituierten Alkansulfonsäuren mit 1 bis 4 Kohlenstoffatomen oder auch mit Benzol- oder Naphthalinsulfonsäuren, welche gegebenenfalls durch Fluor, Chlor, Brom oder Methyl substituiert sind.

Verwendet man beispielsweise für die Verfahrensvariante (a) N'-(4,6-Dimethoxy-pyrimidin-2-yl)-N''-benzyloxy-N'''-(2-chlor-phenylmethylsulfonyl)-guanidin und Trifluoressigsäureanhydrid als Ausgangsstoffe, so kann der Reaktionsablauf durch das folgende Formelschema skizziert werden:

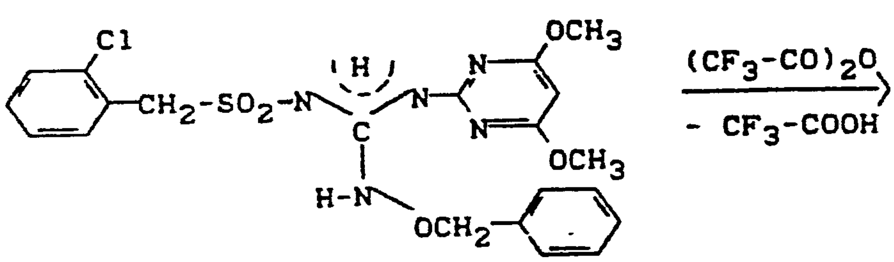

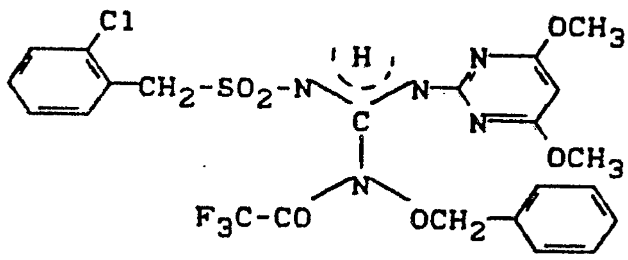

Verwendet man beispielsweise für die Verfahrensvariante (b) N'-(4-Ethoxy-6-methyl-s-triazin-2-yl)-N''-methoxy-N'''-(2-ethoxycarbonyl-benzolsulfonyl)-guanidin und 2-Chlor-phenylisocyanat als Ausgangsstoffe, so kann der Reaktionsablauf durch das folgende Formelschema skizziert werden:

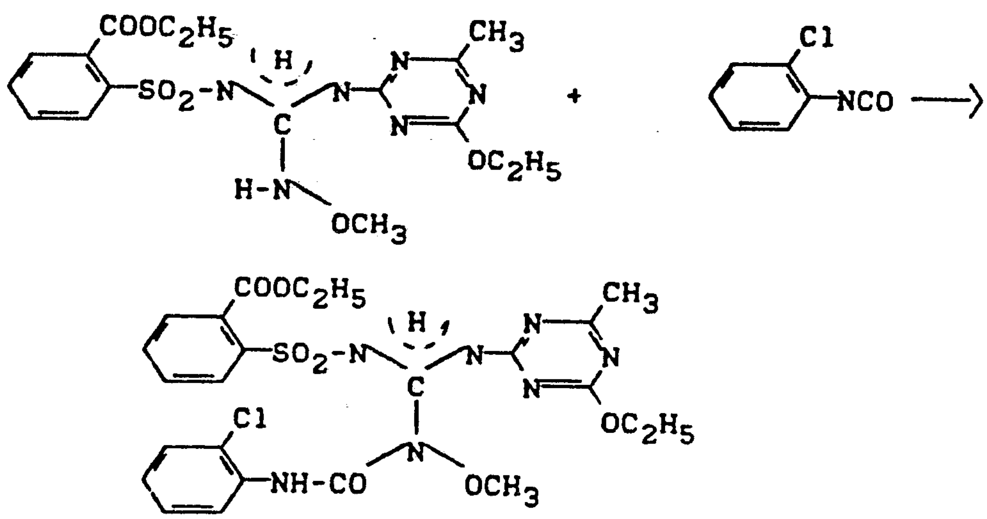

Verwendet man beispielsweise für die Verfahrensvariante (c) N'-(4-Methoxy-6-methyl-pyrimidin-2-yl)-N''-methoxy-N''-(4-fluor-benzoyl)-guanidin und 2-Methoxycarbonyl-benzolsulfonsäurechlorid als Ausgangsstoffe, so kann der Reaktionsablauf durch das folgende Formelschema skizziert werden:

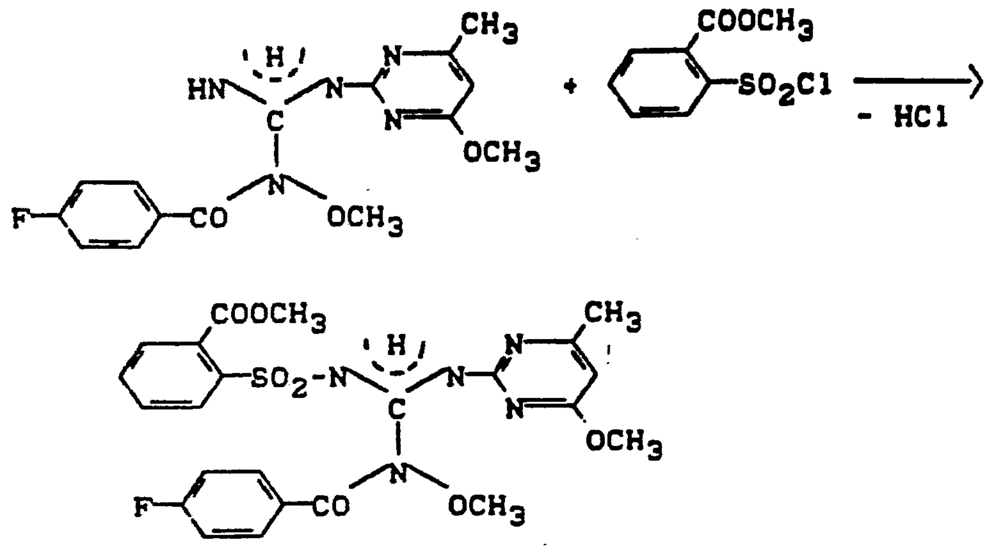


Verwendet man beispielsweise für die Verfahrensvariante (d) N'-(4-Methoxy-6-methyl-s-triazin-2-yl)-N''-isopropoxy-N''-methylcarbonyl-N'''-(2-methoxycarbonyl-benzolsulfonyl)-guanidin und Natriummethylat als Ausgangsstoffe, so kann der Reaktionsablauf durch das folgende Formelschema skizziert werden:

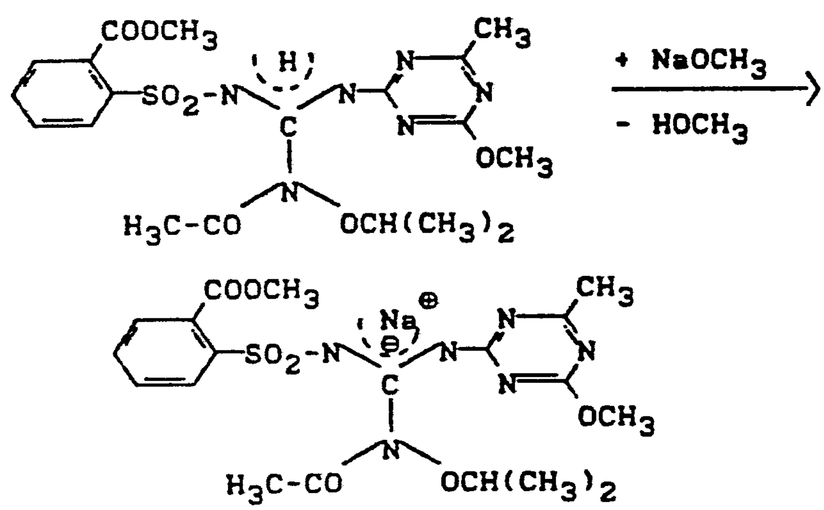

Verwendet man beispielsweise für die Verfahrensvariante (e) N'-(4-Difluormethoxy-6-methyl-pyrimidin-2-yl)-N''-ethoxy-N''(1-chlor-1-ethyl)-carbonyl-N'''-(2-chlorbenzolsulfonyl)-guanidin und Methansulfonsäure als Ausgangsstoffe, so kann der Reaktionsablauf durch folgendes Formelschema skizziert werden:

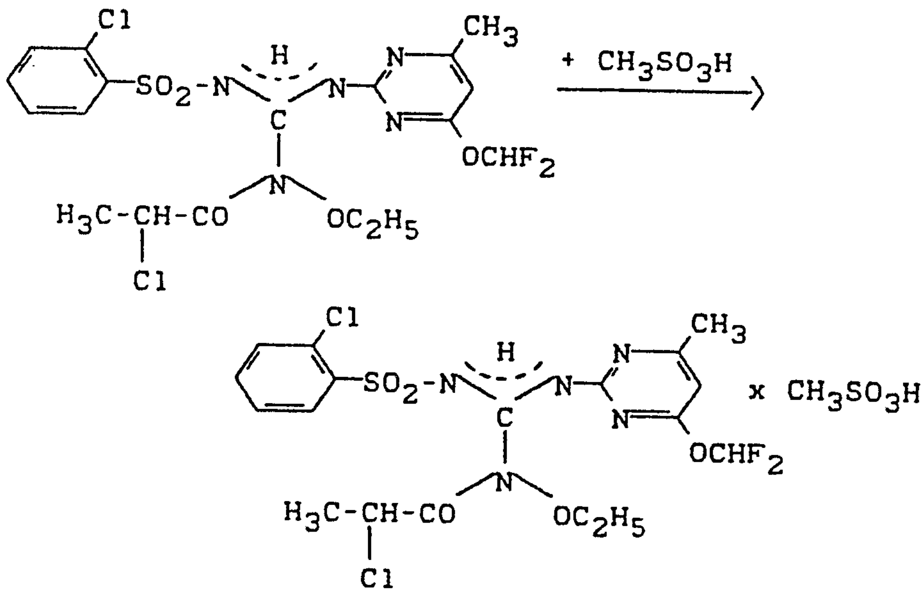


Verwendet man beispielsweise für die Verfahrensvariante (f/α) N'-(4-Methoxy-6-methyl-s-triazin-2-yl)-N''-isopropoxy-N''-ethylcarbonyl-N'''-(2-methoxycarbonyl-benzolsulfonyl)-guanidin und Acetylchlorid als Ausgangsstoffe, so kann der Reaktionsablauf durch das folgende Formelschema skizziert werden:

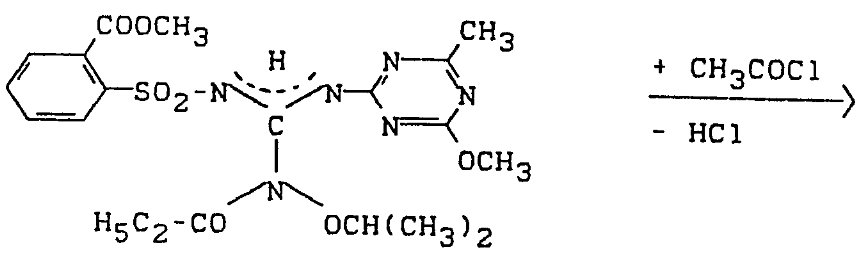

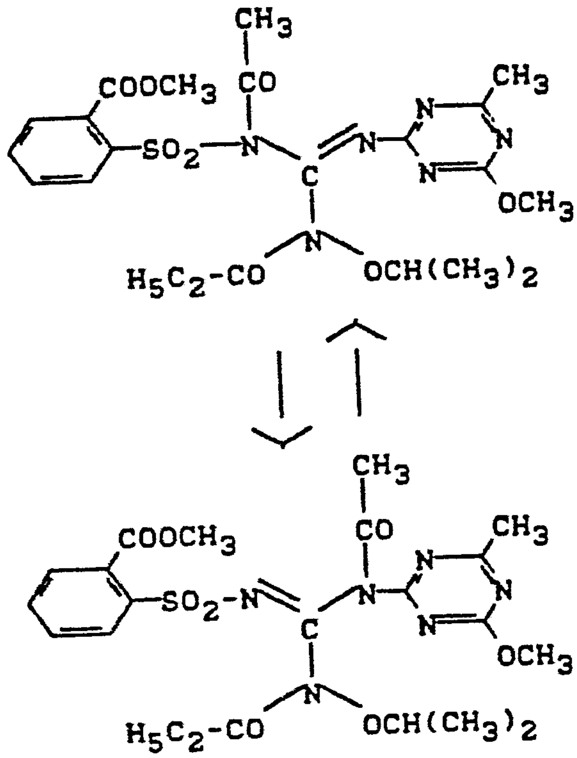

Verwendet man beispielsweise für die Verfahrensvariante (f/β) N'-(4-Methoxy-6-methyl-pyrimidin-2-yl)-N''-methoxy-N'''-(2-methoxycarbonyl-benzolsulfonyl)-guanidin und mindestens die zweifache molare Menge Acetylchlorid als Ausgangsstoffe, so kann der Reaktionsablauf durch das folgende Formelschema skizziert werden:

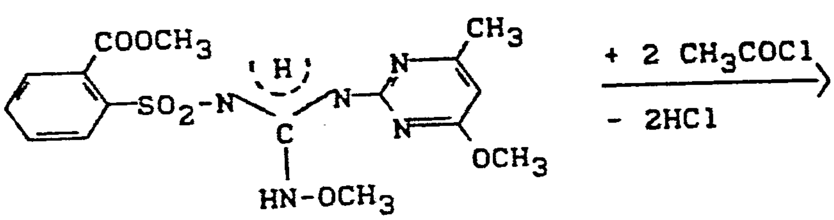

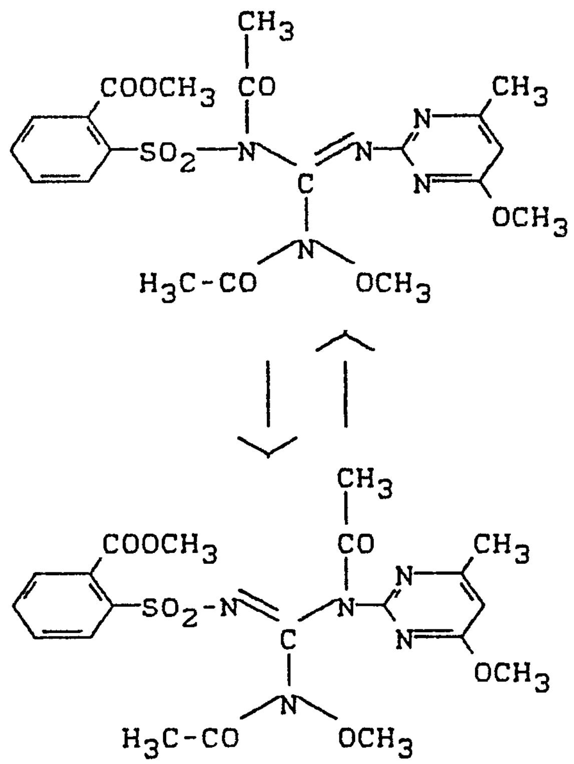

Die bei der Verfahrensvariante (a) als Ausgangsstoffe zu verwendenden Sulfonylguanidin-Derivate sind durch die Formel (II) allgemein definiert. In Formel (II) haben X, Y, Z, $R^1$, $R^2$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (II) seien genannt:
N'-(4,6-Dimethyl-pyrimidin-2-yl)-, N'-(4,6-Dimethoxy-pyrimidin-2-yl)-, N'-(4-Methoxy-6-methyl-pyrimidin-2-yl)-, N'-(4-Ethoxy-6-methyl-pyrimidin-2-yl)-, N'-(4-Methyl-pyrimidin-2-yl)-, N'-(4-Chlor-6-methoxy-pyrimidin-2-yl)-, N'-(4-Chlor-6-ethoxy-pyrimidin-2-yl)-, N'-(4-Chlor-6-dimethylamino-pyrimidin-2-yl)-, N'-(4-Methyl-6-methylthio-pyrimidin-2-yl)-, N'-(4-Dimethylamino-6-methyl-pyrimidin-2-yl)-, N'-(4-Difluormethoxy-6-methyl-pyrimidin-2-yl)-, N'-(2,6-Dimethyl-pyrimidin-4-yl)-, N'-(2,6-Dimethoxy-pyrimidin-4-yl)-, N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-, N'-(4-Diethylamino-6-ethylthio-s-triazin-2-yl)-, N'-(5,6-Dimethyl-1,2,4-triazin-3-yl)-,

N'-(5-Methyl-1,2,4-triazin-3-yl)- und N'-(4,6-Dimethyl-pyrid-2-yl)-N"-methoxy-, -N"-ethoxy-, -N"-propoxy-, -N"-isopropoxy-, -N"-butoxy-, -N"-isobutoxy-, -N"-s.-butoxy-, -N"-pentoxy-, -N"-hexyloxy-, -N"-octyloxy-, -N"-allyloxy-, -N"-crotyloxy-, -N"-(3-chlor-propoxy)-, -N"-methoxycarbonylmethoxy-, -N"-ethoxycarbonylmethoxy, -N"-(1-methoxycarbonylethoxy)-, -N"-(1-ethoxycarbonyl-ethoxy)-, -N"-(2-phenylethoxy)-, -N"-phenoxy-, -N"-benzyloxy-, -N"-(4-methyl-benzyloxy)-, N"-(4-fluor-benzyloxy)-, -N"-(4-chlor-benzyloxy)-, -N"-(4-nitro-benzyloxy)-, -N"-(2,6-dichlor-benzyloxy)-, -N"-(4-methoxycarbonylbenzyloxy)- und -N"-(4-ethoxycarbonyl-benzyloxy)-N'''-benzolsulfonyl-, -N'''-(2-chlor-benzolsulfonyl)-, -N'''-(3-chlor-benzolsulfonyl)-, -N'''-(4-chlor-benzolsulfonyl)-, -N'''-(2-fluor-benzolsulfonyl)-, -N'''-(4-fluor-benzolsulfonyl)-, -N'''-(2-brom-benzolsulfonyl)-, -N'''-(4-brombenzolsulfonyl)-, -N'''-(2-cyano-benzolsulfonyl)-, -N'''-(2-nitro-benzolsulfonyl)-, -N'''-(4-nitro-benzolsulfonyl)-, -N'''-(2-methyl-benzolsulfonyl)-N'''-(4-methyl-benzolsulfonyl)-, -N'''-(2-chlormethylbenzolsulfonyl)-, -N'''-(2-trifluormethyl-benzolsulfonyl)-, -N'''-(2-methoxy-benzolsulfonyl)-, -N'''-(4-methoxy-benzolsulfonyl)-, -N'''-(2-methylthio-benzolsulfonyl)-, -N'''-(2-difluormethoxybenzolsulfonyl)-, -N'''-(2-trifluormethoxy-benzolsulfonyl)-, -N'''-(2-methylthiomethyl-benzolsulfonyl)-, -N'''-(2-dimethylaminosulfonyl-benzolsulfonyl)-, -N'''-(2-phenyl-benzolsulfonyl)-,-N'''-(2-methoxysulfonyl-benzolsulfonyl)-, -N'''-(2-methoxycarbonyl-benzolsulfonyl)-, -N'''-(2-ethoxycarbonyl-benzolsulfonyl)-, -N'''-(2-propoxycarbonyl-benzolsulfonyl)-, -N'''-(2-methylaminocarbonylbenzolsulfonyl)-, -N'''-(2-ethylaminocarbonyl-benzolsulfonyl)-, -N'''-(2-propylaminocarbo-

nyl-benzolsulfonyl)-, -N'''-(2-methoxyaminocarbonyl-benzolsulfonyl)-, -N'''-(2-ethoxyaminocarbonyl-benzolsulfonyl)-, -N'''-(2-propoxyaminocarbonyl-benzolsulfonyl)-, -N'''-(2-dimethylaminocarbonyl-benzolsulfonyl)-, -N'''-(2-diethylaminocarbonyl-benzolsulfonyl)- und -N'''-(2-methoxyaminosulfonyl-benzolsulfonyl)-guanidin.

Die Verbindungen der Formel (II) sind bekannt und/oder lassen sich nach bekannten Verfahren herstellen (vergl. z. B. EP-A 121 082).

Die beim erfindungsgemäßen Verfahren (a) außerdem als Ausgangsstoffe zu verwendenden Acylierungsmittel sind durch die Formel (III) allgemein definiert. In dieser Formel (III ) steht $R^7$ vorzugsweise für diejenigen Reste, die bereits im Zusammenhang mit der Beschreibung der erfindungsgemäßen Stoffe der Formel (I) vorzugsweise für diesen Substituenten genannt wurden. W steht in dieser Formel vorzugsweise für Halogen wie Fluor, Chlor, Brom oder für den Rest $R^7COO$-.

Als Beispiele für die Verbindungen der Formel (III) seien genannt:

$$R^7COW \qquad (III)$$

W = Fluor, Chlor, Brom oder $R^7COO$-

Tabelle 1

| $R^7$ | $R^7$ | $R^7$ |
|---|---|---|
| $CH_3$ | 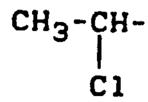 | 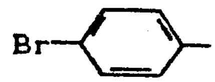 |
| $C_2H_5$ | $ClCH_2-$ | 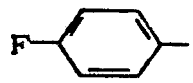 |
| $n-C_3H_7$ | $CH_3O-CH_2-$ | 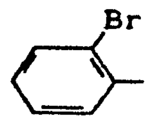 |
| $i-C_3H_7$ | 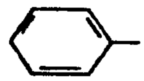 | 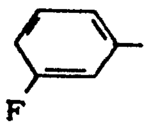 |
| $n-C_4H_9$ | 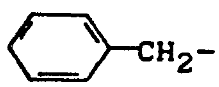 | 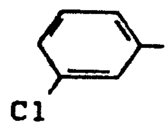 |
| $i-C_4H_9$ | 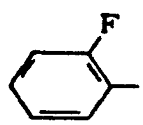 | 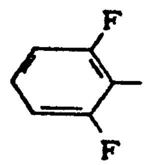 |
| $s-C_4H_9$ | 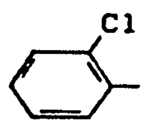 | 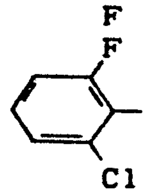 |
| $t-C_4H_9$ | 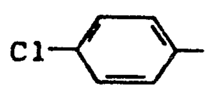 | 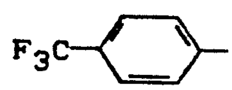 |
| $CF_3-$ | 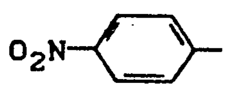 | 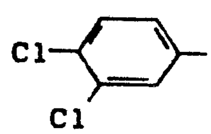 |
| $CHCl_2-$ | 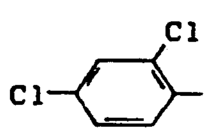 | 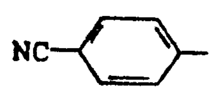 |
| $CCl_3-$ | | |

Die Verbindungen der Formel (III) sind bekannt und/oder lassen sich nach allgemein bekannten Methoden herstellen.

Die bei der Verfhrensvariante (b) als Ausgangsstoffe zu verwendenden Sulfonylguanidin-Derivate sind durch die Formel (II) allgemein definiert. In Formel (II) haben X, Y, Z, $R^1$, $R^2$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Verbindungen der Formel (II) wurden bereits oben bei der Beschreibung der Ausgangsstoffe für das Verfahren (a) genannt.

Die beim erfindungsgemäßen Verfahren (b) außerdem als Ausgangsstoffe zu verwendenden Isocyanate sind durch die Formel (IV) allgemein definiert. In dieser Formel (IV) steht $R^8$ vorzugsweise für diejenigen Reste, die bereits im Zusammenhang mit der Beschreibung der erfindungsgemäßen Stoffe der Formel (I) vorzugsweise für diesen Substituenten genannt wurden.

Als Beispiele für die Verbindungen der Formel (IV) seien genannt:

$$R^8\text{-NCO} \qquad (IV)$$

Tabelle 2

| $R^8$ | $R^8$ | $R^8$ |
|---|---|---|
| $CH_3$- | 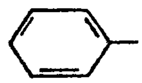 | 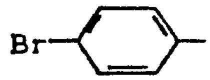 |
| $C_2H_5$- | 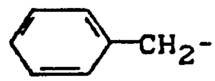 | 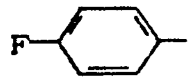 |
| n-$C_3H_7$- | 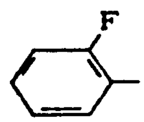 | 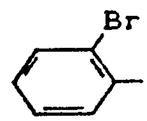 |
| $(CH_3)_2CH$- | 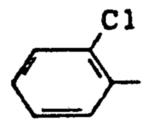 | 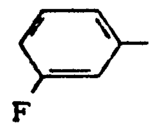 |
| 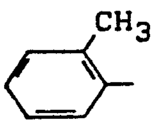 | 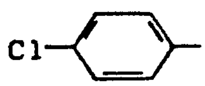 | 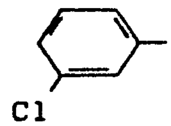 |
| 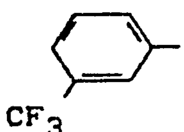 | 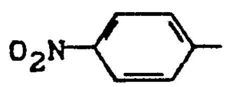 | 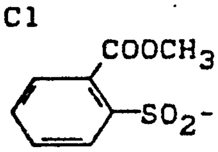 |
| 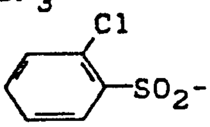 | 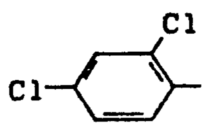 | 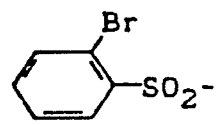 |
| 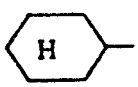 | 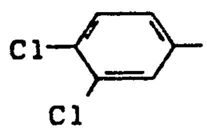 | 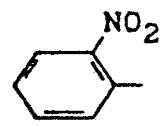 |
| 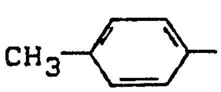 | 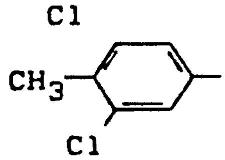 | 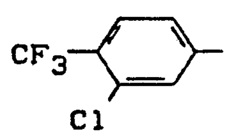 |
| 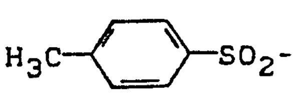 | | |

Die Verbindungen der Formel (IV) sind allgemein bekannte Verbindungen der organischen Chemie.

Die bei der Verfahrensvariante (c) als Ausgangsstoffe zu verwendenden Guanidin-Derivate sind durch die Formel (V) allgemein definiert. In Formel (V) haben X, Y, Z, $R^2$, $R^3$ und $R^4$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (V) seien beispielsweise genannt:

N'-(2,6-Dimethoxy-pyrimidin-4-yl)-, N'-(2,6-Dimethyl-pyrimidin-4-yl)-, N'-(4,6-Dimethyl-pyrimidin-2-yl)-, N'-(4-Methyl-pyrimidin-2-yl)-, N'-(4-Ethyl-pyrimidin-2yl)-, N'-(4-Methoxy-6-methyl-pyrimidin-2-yl)-, N'-(4-Ethoxy-6-methyl-pyrimidin-2-yl)-, N'-(4-Propoxy-6-methyl-2-yl)-, N'-(4-Isopropoxy-6-methyl-pyrimidin-2-yl)-, N'-(4-Chlor-6-methoxy-pyrimidin-2-yl)-, N'-(4-Chlor-6-ethoxy-pyrimidin-2-yl)-, N'-(4-Chlor-6-dimethylamino-pyrimidin-2-yl)-, N'-(4-Methyl-6-methylthio-pyrimidin-2-yl)-, N'-(4,6-Dimethoxy-pyrimidin-2-yl)-, N'-(4-Difluormethoxy-6-methyl-pyrimidin-2-yl)-, N'-(4-Dimethylamino-6-methyl-pyrimidin-2-yl)-, N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-

s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-4,6-(Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-, N'-(4-Diethylamino-6-ethylthio-s-triazin-2-yl)-, N'-(5,6-Dimethyl-1,2,4-triazin-3-yl)-, N'-(5-Methyl-1,2,4-triazin-3-yl)- und N'-(4,6-Dimethyl-pyrid-2-yl)-N''-methylcarbonyl-, -N''-methylaminocarbonyl-, -N''-phenylcarbonyl-, -N''-phenylaminocarbonyl-, -N''-(4-chlor-phenyl)-carbonyl-, -N''-(4-chlor-phenyl)-aminocarbonyl-, -N''-(4-fluor-phenyl)-carbonyl-, -N''-(4-fluor-phenyl)-aminocarbonyl-, -N''-(4-brom-phenyl)-carbonyl-, -N''-(4-brom-phenyl)-aminocarbonyl-, -N''-ethylcarbonyl-, -N''-n-propylcarbonyl-, -N''-i-propylcarbonyl-, -N''-n-butylcarbonyl-, -N''-i-butylcarbonyl-, -N''-sec.-butylcarbonyl-, -N''-tert.-butylcarbonyl-, -N''-trifluormethylcarbonyl-, -N''-

dichlormethylcarbonyl-, -N''-trichlormethylcarbonyl-, -N''-(1-chlor-1-ethyl)-carbonyl-, -N''-(3-chlor-phenyl)-carbonyl-, -N''-(3-chlor-phenyl)-aminocarbonyl-, -N''-(4-nitro-phenyl)-carbonyl-, -N''-benzylcarbonyl-, -N''-benzylaminocarbonyl-, -N''-(4-methyl-phenylsulfonyl)-aminocarbonyl-, -N''-(2-chlor-phenyl)-carbonyl-, -N''-(2-chlor-phenyl)-aminocarbonyl-, -N''-(2,4-dichlor-phenyl)-carbonyl-, -N''-(2,4-dichlor-phenyl)-aminocarbonyl-, -N''-(2-fluor-phenyl)-carbonyl-, -N''-(2-fluor-phenyl)-aminocarbonyl-, -N''-(3-fluor-phenyl)-carbonyl-, -N''-(3-fluor-phenyl)-aminocarbonyl-, -N''-(3-brom-phenyl)-carbonyl-, -N''-(3-brom-phenyl)-aminocarbonyl-, -N''-(2-brom-phenyl)-carbonyl-, -N''-(2-brom-phenyl)-aminocarbonyl-, -N''-cyclohexylaminocarbonyl-, -N''-(3,4-dichlor-phenyl)-aminocarbonyl-, -N''-isopropylamino-carbonyl-, -N''-(2-nitro-phenyl)-aminocarbonyl-, -N''-(4-nitro-phenyl)-aminocarbonyl- und -N''-(3-trifluormethyl-phenyl)-aminocarbonyl-N"-methoxy-guanidin, -N"-ethoxy-guanidin, -N"-propoxy-guanidin, -N"-isopropoxy-guanidin, -N"-butoxy-guanidin, -N"-isobutoxy-guanidin, -N"-sec.-butoxy-guanidin, -N"-pentoxy-guanidin, -N"-isopentoxy-guanidin, -N"-hexyloxy-guanidin, -N"-octyloxy-guanidin, -N"-allyloxy-guanidin, -N"-(2-chlor-ethoxy)-guanidin, -N"-(2-fluor-ethoxy)-guanidin, -N"-(2-chlor-propoxy)-guanidin, -N"-(2-fluor-propoxy)-guanidin, -N"-(3-chlor-propoxy)-guanidin, -N"-(4-chlor-butoxy)-guanidin, -N"-methoxycarbonylmethoxy-guanidin, -N"-ethoxycarbonylmethoxy-guanidin, -N"-(1-methoxy-carbonyl-ethoxy)-guanidin, -N"-(1-ethoxycarbonyl-ethoxy)-guanidin, -N"-dimethylamino-carbonylmethoxy-guanidin, -N"-(2-phenyl-ethoxy)-guanidin, -N"-phenoxy-guanidin, -N"-(4-methylbenzyloxy)-guanidin, -N"-(4-fluorbenzyloxy)-guanidin, -N"-(4-chlor-benzyloxy)-guanidin, -N"-(4-nitro-benzyloxy)-guanidin, -N"-(2,6-dichlor-benzyloxy)-guanidin, -N"-(4-methoxycarbonylbenzyloxy)-guanidin und -N"-(4-ethoxycarbonyl-benzyloxy)-guanidin.

Die als Ausgangsstoffe zu verwendenden Guanidin-Derivate der Formel (V) sind neu. Man erhält die Guanidin-Derivate der Formel (V), wenn man Guanidine der Formel (VII)

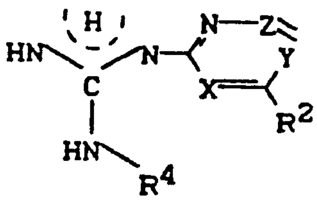

(VII)

in welcher

X, Y, Z, $R^2$ und $R^4$ die oben angegebenen Bedeutungen haben,

(a) für den Fall, daß $R^3$ für den Rest $R^7$ steht, mit Acylierungsmitteln der Formel (III)

$R^7COW$ (III)

in welcher

$R^7$ und W die oben angegebenen Bedeutungen haben,

gegebenenfalls in Gegenwart von Säureakzeptoren wie z. B. 1,4-Diazabicyclo-[2,2,0]-octan und Pyridin und gegebenenfalls in Gegenwart von inerten Verdünnungsmitteln wie z. B. Methylenchlorid, Chloroform und Dioxan, bei Temperaturen zwischen -20 °C und +100 °C, vorzugsweise zwischen -5 °C und +80 °C umsetzt (die Aufarbeitung der Reaktionsprodukte der Formel (V) kann auf übliche Weise erfolgen); oder

(b) für den Fall, daß $R^3$ für den Rest $-NHR^8$ steht, mit Isocyanaten de Formel (IV)

$$R^8\text{-NCO} \qquad \text{(IV)}$$

in welcher

$R^8$ die oben angegebenen Bedeutungen hat,

gegebenenfalls in Gegenwart von Katalysatoren wie z. B. organische Zinn-Verbindungen wie Dibutylzinndilaurat oder tertiäre Amine wie z. B. Triethylamin und 1,4-Diazabicyclo-[2,2,2]-octan und gegebenenfalls in Gegenwart von inerten Verdünnungsmitteln wie z. B. Toluol, Dioxan, Dimethylformamid und Acetonitril bei Temperaturen zwischen 0 °C und 140 °C, vorzugsweise zwischen 10 °C und 120 °C umsetzt. Die Aufarbeitung der Reaktionsprodukte der Formel (V) kann auf übliche Weise erfolgen.

Die Guanidine der Formel (VII) sind bekannt und/oder lassen sich nach bekannten Methoden herstellen (vergl. z. B. EP-A 121 082). Die Ausgangsprodukte der Formeln (III) und (IV) sind bekannte Verbindungen der organischen Chemie.

Die für das Verfahren (c) weiterhin zu verwendenden Sulfonsäurechloride sind durch die Formel (VI) allgemein definiert. In Formel (VI) hat $R^1$ vorzugsweise bzw. insbesondere die gleichen Bedeutungen wie sie oben im Rahmen der Substituentendefinition der Formel (I) vorzugsweise bzw. als insbesondere bevorzugt angegeben sind.

Als Ausgangsstoffe der Formel (VI) seien beispielsweise genannt:
2-Chlor-, 3-Chlor-, 4-Chlor-, 2,5-Dichlor-, 2-Fluor-, 4-Fluor-, 2-Brom-, 4-Brom-, 2-Nitro-, 4-Nitro-, 2-Cyano-, 2-Methyl-, 4-Methyl-, 2-Chlormethyl-, 2-Trifluormethyl-, 2-Methoxy-, 2-Difluormethoxy-, 2-Trifluormethoxy-, 2-Methylthio-, 2-Methylthiomethyl-, 2-Methylsulfinylmethyl-, 2-Methylsulfonylmethyl-, 2-Dimethylaminosulfonyl-, 2-Diethylaminosulfonyl-, 2-Phenyl-, 2-Methoxycarbonyl-, 2-Ethoxycarbonyl-, 2-Propoxycarbonyl-, 2-Isopropoxycarbonyl-, 2-Butoxycarbonyl-, 2-Dimethylaminocarbonyl-, 2-Diethylaminocarbonyl-, 2-Phenoxy-, 2-Methyl-5-chlor-, 2-Chlor-5-trifluormethyl-, 2-Methylsulfonyl-, 2-Ethylsulfonyl-, 2-Chlor-4-trifluormethoxy-, 3-Chlor-4-trifluormethoxy-, 2-Trifluormethoxy-5-chlor-und 3,5-Dichlor-benzolsulfonsäurechlorid; (2-Chlor-phenyl)-, (2-Cyano-phenyl)- und (2-Methoxy-carbonyl-phenyl)-methansulfonsäurechlorid; ferner Methan-, Chlormethan-, Trifluormethan-, Ethan-, 2-Chlorethan-, Ethen-, Propan-, Butan-, Perfluorbutan- und Perfluoroctan-sulfonsäurechlorid.

Die Sulfonsäurechloride der Formel (VI) sind bekannt und/oder können nach an sich bekannten Verfahren hergestellt werden (vgl. J. Org. Chem. 33 (1968), 2104; J. Org.

Chem. 25 (1960), 1824; DE-AS 2 308 262; EP-OS 23 140, 23 141, 23 422, 35 893, 48 143, 51 466, 64 322, 70 041, 44 808, 44 809; US-PS 2 929 820, 4 282 242, 4 348 220 und 4 372 778 sowie Angew. Chem. 93 (1981), 151).

Die bei der Verfahrensvariante (d) als Ausgangsstoffe zu verwendenden Verbindungen sind durch die Formel (I) - mit der Maßgabe, daß M für Wasserstoff steht - allgemein definiert. In Formel (I) – soweit sie die als Ausgangsstoffe für Verfahren (d) zu verwendenden Verbindungen betrifft – steht M für Wasserstoff und X, Y, Z, $R^1$, $R^2$, $R^3$ und $R^4$ haben vorzugsweise bzw. insbesondere die gleichen Bedeutungen, wie sie oben im Rahmen der Substituentendefinition der Formel (I) als bevorzugt bzw. als insbesondere bevorzugt angegeben sind.

Die als Ausgangsstoffe für Verfahren (d) zu verwendenden Verbindungen der Formel (I) können nach den unter (a), (b) und (c) beschriebenen Verfahren hergestellt werden.

Als Beispiele für die bei Verfahren (d) zu verwendenden Metallhydroxide, -hydride, -alkanolate bzw. metallorganischen Verbindungen seien genannt: Lithium-, Natrium-, Kalium-, Magnesium- und Calcium-hydroxid, Lithium-, Natrium- und Calcium-hydrid, Natrium-methanolat und -ethanolat, Kalium-methanolat, -ethanolat und Kalium-tert.-butanolat sowie Butyllithium und Isopropylmagnesiumchlorid.

Die bei der Verfahrensvariante (e) als Ausgangsstoffe zu verwendenden Verbindungen sind durch die Formel (I) - mit

der Maßgabe, daß M für Wasserstoff steht - allgemein definiert. In Formel (I) — soweit sie die als Ausgangsstoffe für Verfahren (e) zu verwendenden Verbindungen betrifft — steht M für Wasserstoff und X, Y, Z, $R^1$, $R^2$, $R^3$ und $R^4$ haben vorzugsweise bzw. insbesondere die gleichen Bedeutungen, wie sie oben im Rahmen der Substituentendefinition der Formel (I) vorzugsweise bzw. als insbesondere bevorzugt angegeben sind.

Die als Ausgangsstoffe für Verfahren (e) zu verwendenden Verbindungen der Formel (I) können nach den unter (a), (b) und (c) beschriebenen Verfahren hergestellt werden.

Bei Verfahren (e) werden starke Säuren als Ausgangsstoffe eingesetzt. Vorzugsweise sind dies Halogenwasserstoffsäuren, wie Hydrogenchlorid, Hydrogenbromid oder Hydrogeniodid, weiter Schwefelsäure oder gegebenenfalls durch Fluor oder Chlor substituierte Alkansulfonsäuren mit bis zu 4 Kohlenstoffatomen wie z. B. Methansulfonsäure, Ethansulfonsäure, Chlormethansulfonsäure, 2-Chlorethansulfonsäure und Trifluormethansulfonsäure, Trifluoressigsäure, ferner Benzolsulfonsäure, p-Toluolsulfonsäure, Naphthalin-1-sulfonsäure, Naphthalin-2-sulfonsäure, Naphthalin-1,4-, -1,5- -1,6-, -2,6- und -2,7-disulfonsäure.

Die bei der Verfahrensvariante (f-α) als Ausgangsstoffe zu verwendenden Verbindungen sind durch die Formel (I) - mit der Maßgabe, daß M für Wasserstoff steht - allgemein definiert. In Formel (I) - soweit sie die als Ausgangsstoffe für Verfahren (f-α) zu verwendenden Verbindungen betrifft - steht M für Wasserstoff und X, Y, Z, $R^1$, $R^2$,

$R^3$ und $R^4$ haben vorzugsweise bzw. insbesondere die gleichen Bedeutungen wie sie oben im Rahmen der Substituentendefinition der Formel (I) als bevorzugt bzw. als insbesondere bevorzugt angegeben sind.

Die als Ausgangsstoffe für Verfahren (f-α) zu verwendenden Verbindungen der Formel (I) können nach den unter (a), (b) und (c) beschriebenen Verfahren hergestellt werden.

Die beim erfindungsgemäßen Verfahren (f-β) als Ausgangsstoffe zu verwendenden Sulfonylguanidin-Derivate sind durch die Formel (II) allgemein definiert. In dieser Formel (II) stehen X, Y, Z, $R^1$, $R^2$ und $R^4$ vorzugsweise für diejenigen Reste, die bereits im Zusammenhang mit der Beschreibung der erfindungsgemäßen Stoffe der Formel (I) vorzugsweise für diese Substituenten genannt wurden.

Beispiele für die Sulfonylguanidin-Derivate der Formel (II) und ihre Herstellungsverfahren wurden bereits weiter oben im Zusammenhang mit der Beschreibung der Ausgangsstoffe für Verfahren (a) angegeben.

Die bei den Verfahrensvarianten (f-α) und (f-β) weiter als Ausgangsstoffe zu verwendenden Acylierungsmittel sind durch die Formel (III) allgemein definiert. In Formel (III) hat $R^7$ vorzugsweise bzw. insbesondere die gleiche Bedeutung, wie sie oben im Rahmen der Substituentendefinition der Formel (I) vorzugsweise bzw. als insbesondere bevorzugt genannt ist. W steht in dieser Formel vorzugsweise für Halogen wie Fluor, Chlor, Brom oder für den Rest $R^7COO-$.

Beispiele für die Acylierungsmittel der Formel (III) und ihre Herstellungsverfahren wurden bereits weiter oben im Zusammenhang mit der Beschreibung der Ausgangsstoffe für Verfahren (a) angegeben.

Das erfindungsgemäße Verfahren (a) zur Herstellung der neuen acylierten Sulfonylguanidine der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Dimethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon, Pyridin und 2-Methyl-5-ethylpyridin.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU), 1,4-Diazabicyclo-[2,2,2]-octan (DABCO), 2-Methyl-5-ethyl-pyridin und 4-Dimethylaminopyridin.

Die Reaktionstemperaturen können bei dem erfindungsgemäßen Verfahren (a) in einem größeren Bereich variiert werden. Im allgemeinen arbeitet man bei Temperaturen zwischen -20 °C und +120 °C, vorzugsweise bei Temperaturen zwischen -10 °C und +100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden. Gegebenenfalls nach Abdestillieren flüchtiger Komponenten wird mit Wasser gewaschen, getrocknet, filtriert und eingeengt. Die im Rückstand verbleibenden Produkte der Formel (I) werden durch Digerieren mit organischen Lösungsmitteln wie z. B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen, vorzugsweise jedoch aprotisch polare Solventien in Betracht wie sie oben im Zusammenhang mit der Beschreibung des erfindungsgemäßen Verfahrens (a) genannt wurden.

Als Katalysatoren können für die Umsetzung gemäß Verfahrensvariante (b) vorzugsweise tertiäre Amine wie Triethylamin und 1,4-Diazabicyclo-[2,2,2]-octan sowie organische Zinn-Verbindungen wie z. B. Dibutylzinndilaurat verwendet werden. Der Zusatz solcher Katalysatoren ist jedoch nicht erforderlich.

Die Reaktionstemperaturen können bei dem erfindungsgemäßen Verfahren (b) in einem größeren Bereich variiert werden. Im allgemeinen arbeitet man bei Temperaturen zwischen 0 °C und 140 °C, vorzugsweise bei Temperaturen zwischen 0 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch

auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden. Soweit die Produkte der Formel (I) aus dem Reaktionsgemisch kristallin anfallen, können sie durch Absaugen isoliert werden. Andernfalls wird - gegebenenfalls nach Einengen - mit Wasser verdünnt und mit einem mit Wasser praktisch nicht mischbaren Lösungsmittel wie z. B. Methylenchlorid, extrahiert. Durch Waschen der Extraktionslösung mit Wasser, Trocknen, Filtrieren, Einengen des Filtrats und Umkristallisieren des Rückstandes können die Produkte der Formel (I) in reiner Form erhalten werden.

Das erfindungsgemäße Verfahren (c) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (c) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (c) innerhalb eines größeren Bereiches variiert werden. Im allgemeinen arbeitet man zwischen -80 °C und +100 °C, vorzugsweise zwischen -30 °C und +50 °C. Das Verfahren (c) wird im allgemeinen bei Normaldruck durchgeführt.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem kleineren Überschuß zu verwenden.

Die Durchführung und Aufarbeitung kann bei Verfahren (c) analog zu Verfahren (a) erfolgen.

Das erfindungsgemäße Verfahren (d) wird vorzugsweise unter Verwendung von Verdünnungsmitteln durchgeführt. Als solche kommen praktisch alle inerten organischen Lösungsmittel in Betracht. Hierzu gehören insbesondere Alkohole wie z. B. Ethanol, n- und iso-Propanol, Ether wie z. B. Tetrahydrofuran, Dioxan und 1,2-Dimethoxyethan, Ester wie z.B. Essigsäureethylester und -methylester sowie Nitrile wie z. B. Acetonitril.

Die Reaktionstemperatur kann bei Verfahren (d) innerhalb eines größeren Bereiches variiert werden. Im allgemeinen arbeitet man zwischen -20 °C und +50 °C, vorzugsweise zwischen 0 °C und +30 °C. Das Verfahren (d) wird im allgemeinen bei Normaldruck durchgeführt.

Zur Durchführung des erfindungsgemäßenVerfahrens (d) setzt

man je Mol Verbindung der Formel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (e) wird vorzugsweise unter Verwendung von Verdünnungsmitteln durchgeführt. Als solche kommen praktisch alle inerten organischen Lösungsmittel in Betracht. Hierzu gehören insbesondere Alkohole wie z. B. Methanol, Ethanol, n- und iso-Propanol, Ether wie z. B. Tetrahydrofuran, Dioxan und 1,2-Dimethoxyethan, Ester wie z. B. Essigsäuremethylester und -ethylester sowie Ketone wie Aceton, Methylethylketon und Methylisobutylketon.

Soweit die als Ausgangsstoffe verwendeten Säuren in wässriger Lösung eingesetzt werden, kann vorteilhaft auch Essigsäureanhydrid als Verdünnungsmittel verwendet werden.

Die Reaktionstemperatur kann bei Verfahren (e) innerhalb eines größeren Bereiches variiert werden. Im allgemeinen arbeitet man zwischen -20 °C und +50 °C, vorzugsweise zwischen 0 °C und +30 °C. Das Verfahren (e) wird im allgemeinen bei Normaldruck durchgeführt.

Zur Durchführung des erfindungsgemäßen Verfahrens (e) setzt man je Mol Verbindung der Formel (I) im allgemeinen zwischen 1 und 10 Mol, vorzugsweise zwischen 1 und 5 Mol einer starken Säure ein.

Im allgemeinen werden die Verbindungen der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Das erfindungsgemäße Verfahren (f/α) wird vorzugsweise unter Verwendung von Verdünnungsmitteln durchgeführt. Als solche kommen praktisch alle inerten organischen, vorzugsweise jedoch aprotisch polare Solventien in Betracht wie sie oben im Zusammenhang mit der Beschreibung des erfindungsgemäßen Verfahrens (a) genannt wurden.

Als Säureakzeptoren können bei Verfahren (f/α) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (f/α) innerhalb eines größeren Bereiches variiert werden. Im allgemeinen arbeitet man zwischen -20 °C und +120 °C, vorzugsweise zwischen -10 °C und +100 °C. Das Verfahren (f/α) wird im allgemeinen bei Normaldruck durchgeführt.

Zur Durchführung des erfindungsgemäßen Verfahrens (f/α) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (f/α) jeweils nach üblichen Methoden.

Das erfindungsgemäße Verfahren (f/β) wird vorzugsweise unter Verwendung von Verdünnungsmitteln durchgeführt. Als solche kommen praktisch alle inerten organischen, vorzugsweise jedoch aprotisch polare Solventien in Betracht wie sie oben im Zusammenhang mit der Beschreibung des erfindungsgemäßen Verfahrens (a) genannt wurden.

Als Säureakzeptoren können bei Verfahren (f/β) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (f/β) innerhalb eines größeren Bereiches variiert werden. Im allgemeinen arbeitet man zwischen -20 °C und +120 °C, vorzugsweise zwischen -10 °C und +100 °C. Das Verfahren (f/β) wird im allgemeinen bei Normaldruck durchgeführt.

Zur Durchführung des erfindungsgemäßen Verfahrens (f/β) setzt man je Mol Sulfonylguanidin-Derivat der Formel (II) im allgemeinen zwischen 2 und 5, vorzugsweise zwischen 2,5 und 4,0 Mol Acylierungsmittel der Formel (III) und zwischen 2 und 5, vorzugsweise zwischen 2,5 und 4,0 Mol Säureakzeptor ein.

Die Durchführung und Aufarbeitung kann bei Verfahren (f/β) analog zu Verfahren (a) erfolgen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen

sich zur Bekämpfung mono- und dikotyler Unkräuter im Vor- und Nachauflaufverfahren. Sie zeigen insbesondere bei der Anwendung im Nachauflaufverfahren eine gute Kulturpflanzenverträglichkeit für z. B. Weizen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische, pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropyl-phenyl)-1,1-dimethylharnstoff, 2-Chlor-4-ethylamino-6-(1-methylethyl)-1,3,5-triazin, 2-Chlor-4,6-diethylamino-1,3,5-triazin, 2-Ethylamino-6-(1,1-dimethylethyl-amino)-4-methylthio-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4triazin-5(4H)-on, 2-[4,5-Dihydro-4-methyl-4-(1-methylethyl)-5-oxo-1H-imidazol-2-yl]-4(5)-methyl-benzoesäuremethylester, N-Phosphonomethyl-glycin-Derivate, 2-Amino-4-(hydroxymethylphosphinyl)-butansäure, Bis-(1-methyl-4-pyridinium)-dichlorid, 3-(2,4-

Dichlor-phenoxy)-6-nitro-benzoesäuremethylester, 2-[4-(2,4-Dichlorphenoxy)-phenoxy]-propionsäuremethylester, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren

Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

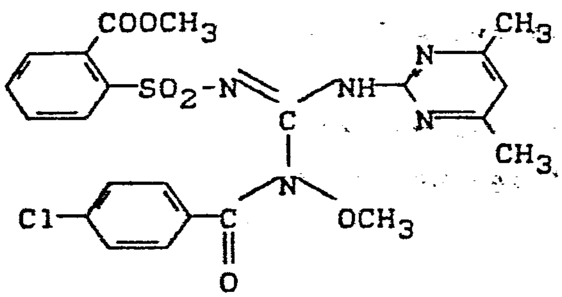

(Verfahren (a))

Eine Lösung von 1,8 g (0,01 Mol) 4-Chlor-benzoylchlorid in 15 ml Methylenchlorid wird unter Rühren zu einer Mischung aus 3,9 g (0,01 Mol) N'-(4,6-Dimethyl-pyrimidin-2-yl)-N''-methoxy-N'''-(2-methoxycarbonyl-benzolsulfonyl)-guanidin, 1,2 g (0,01 Mol) 1,4-Diazabicyclo-[2,2,2]-octan (DABCO) und 20 ml Methylenchlorid gegeben. Die Reaktionsmischung wird 8 Stunden bei 20 °C gerührt, mit Wasser gewaschen, mit Natriumsulfat getrocknet und filtriert. Das Filtrat wird eingeengt und der Rückstand durch Anreiben mit Diethylether zur Kristallisation gebracht.

Man erhält 2,1 g (40 % der Theorie) N'-(4,6-Dimethyl-pyrimidin-2-yl)-N''-methoxy-N''-(4-chlor-benzoyl)-N'''-(2-methoxycarbonyl-benzolsulfonyl)-guanidin vom Schmelzpunkt 132 °C.

Beispiel 2

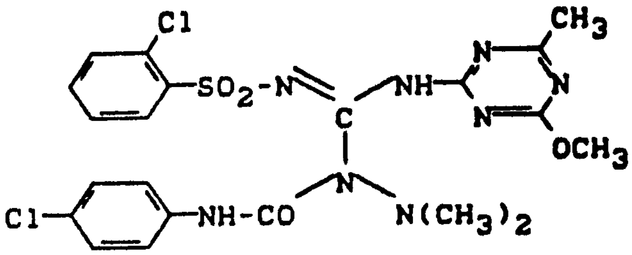

(Verfahren (b))

Eine Mischung aus 2,1 g (0,005 Mol) N'-(4-Methoxy-6-methyl-s-triazin-2-yl)-N''-dimethylamino-N'''-(2-chlor-benzolsulfonyl)-guanidin, 0,9 g (0,005 Mol) 4-Chlor-phenylisocyanat, 0,1 g Dibutylzinnlaurat und 20 ml Dioxan wird 60 Stunden bei 20 °C gerührt. Dann wird das kristallin angefallene Produkt durch Absaugen isoliert.

Man erhält 1,8 g (67 % der Theorie) N'-(4-Methoxy-6-methyl-s-triazin-2-yl)-N''-dimethylamino-N''-(4-chlor-phenylamino-carbonyl)-N'''-(2-chlor-benzol-sulfonyl)-guanidin vom Schmelzpunkt 174 °C.

Beispiel 3

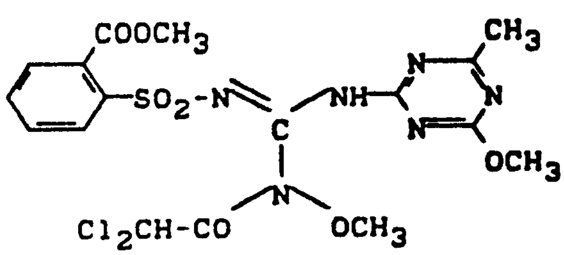

0234250

(Verfahren (c))

7,5 g (0,032 Mol) 2-Chlorsulfonyl-benzoesäure-methylester werden zu einer auf -10 °C gekühlten Mischung aus 9,0 g (0,028 Mol) N'-(4-Methoxy-6-methyl-s-triazin-2-yl)-N''-methoxy-N''-dichloracetyl-guanidin und 100 ml Methylenchlorid gegeben. Unter Rühren werden dann zu dieser auf -10 °C gekühlten Mischung 3,5 g (0,032 Mol) 1,4-Diazabicyclo-[2,2,2]-octan gegeben. Nach 15-stündigem Rühren bei 20 °C wird mit Wasser gewaschen, mit Natriumsulfat getrocknet, filtriert und sorgfältig unter vermindertem Druck eingeengt.

Man erhält 12,9 g (89 % der Theorie) N'-(4-Methoxy-6-methyl-s-triazin-2-yl)-N''-methoxy-N''-dichloracetyl-N'''-(2-methoxycarbonyl-benzolsulfonyl)-guanidin als Öl vom

Brechungsindex $n_D^{20} = 1,5722$.

Beispiel 4

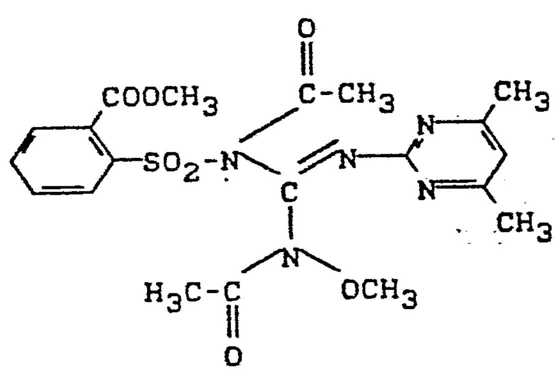

(Verfahren (f/ß)

Eine Lösung von 3,7 g (0,033 Mol) 1,4-Diazabicyclo-[2,2,2]-octan in 30 ml Methylenchlorid wird unter Rühren zu einer Mischung aus 3,9 g (0,01 Mol) N'-(4,6-Dimethyl-pyrimidin-2-yl)-N''-methoxy-N'''-(2-methoxycarbonyl-benzolsulfonyl)-guanidin, 2,3 g (0,03 Mol) Acetylchlorid und 30 ml Methylenchlorid gegeben, wobei eine Innentemperatur zwischen -20 °C und 0 °C eingehalten wird. Die Reaktionsmischung wird dann ohne Kühlung ca. 15 Stunden bei ca. 20 °C gerührt, dann mit Wasser gewaschen, mit Natriumsulfat getrocknet und filtriert. Das Filtrat wird eingeengt und der Rückstand durch Anreiben mit Diethylether zur Kristallisation gebracht.

Man erhält 1,8 g (38 % der Theorie) N'-(4,6-Dimethyl-pyrimidin-2-yl)-N'',N'''-diacetyl-N''-methoxy-N'''-(2-methoxycarbonyl-benzolsulfonyl)-guanidin vom Schmelzpunkt 134 °C.

---

Analog Beispiel 1 bis 4 bzw. Verfahrensvariante (a) bis (f) können die in der nachfolgenden Tabelle 3 aufgeführten Verbindungen der Formel (I) hergestellt werden:

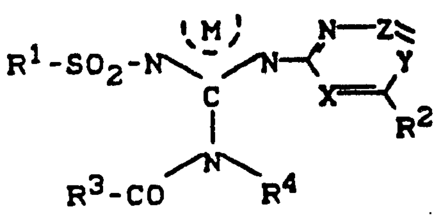

(I)

Den Verbindungen der Formel (I), in welchen M für einen Acylrest $-COR^7$ steht, kann aufgrund ihrer Spektraldaten die allgemeine Struktur (ID) - siehe oben - zugeordnet werden, wie auch in Beispiel 4 geschehen; die isomere Struktur (IC) kann jedoch bisher nicht völlig ausgeschlossen werden.

Tabelle 3

| Beisp.-Nr. | $R^1$ | $R^3$ | $R^4$ | 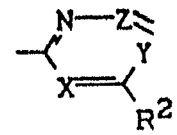 | M | Fp [°C] |
| --- | --- | --- | --- | --- | --- | --- |
| 5 | 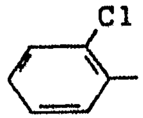 | $CH_3$ | 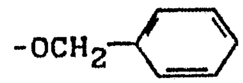 | 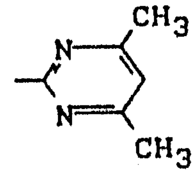 | H | 90 |
| 6 | 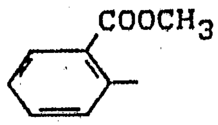 | 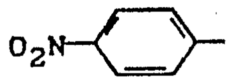 | 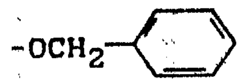 | 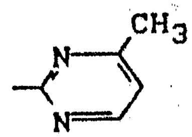 | H | 148 - 149 |
| 7 | 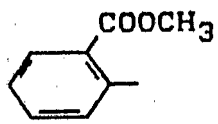 | 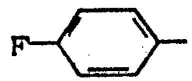 | 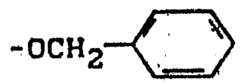 | 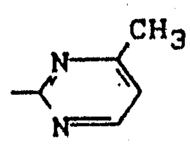 | H | 142 - 145 |
| 8 | 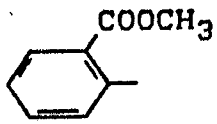 | 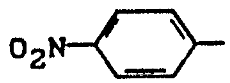 | $-OCH_3$ | 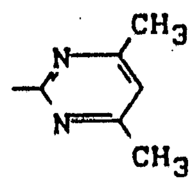 | 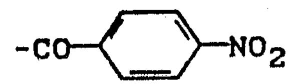 | 201 - 202 |
| 9 | 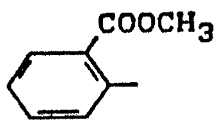 | 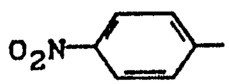 | $-OCH_3$ | 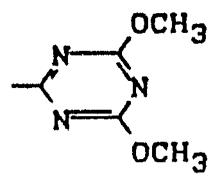 | 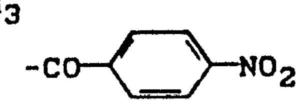 | 113 (Zers.) |

Tabelle 3 - Fortsetzung

| Beisp.-Nr. | $R^1$ | $R^3$ | $R^4$ | 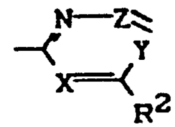 $R^2$ | M | Fp [°C] |
|---|---|---|---|---|---|---|
| 10 | 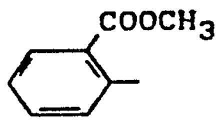 | 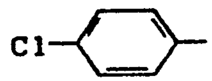 | 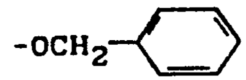 | 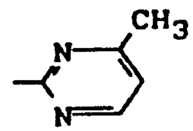 | H | 145 |
| 11 | 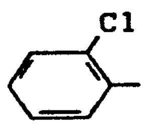 | 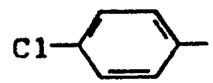 | $-OCH_3$ | 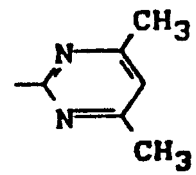 | H | 148 |
| 12 | 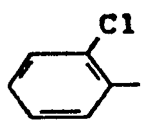 | 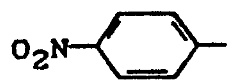 | $-OCH_3$ | 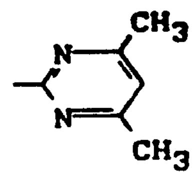 | H | 156 |
| 13 | 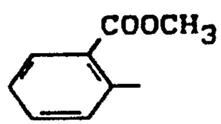 | 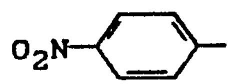 | $-OCH_3$ | 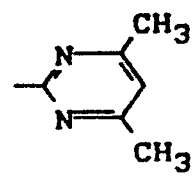 | H | 163 |
| 14 | 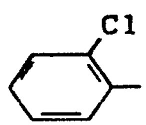 | 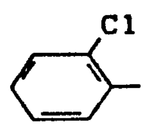 | $-OCH_3$ | 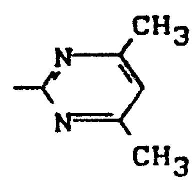 | H | 158 |

Tabelle 3 - Fortsetzung

| Beisp.-Nr. | $R^1$ | $R^3$ | $R^4$ | 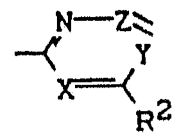 | M | Fp [°C] |
|---|---|---|---|---|---|---|
| 15 | 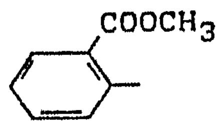 | 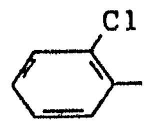 | $-OCH_3$ | 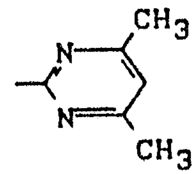 | H | 149 |
| 16 | 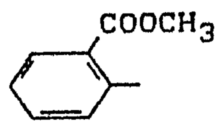 | 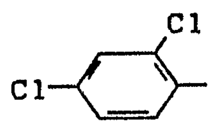 | $-OCH_3$ | 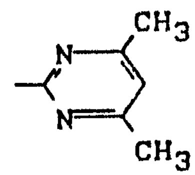 | H | 149 |
| 17 | 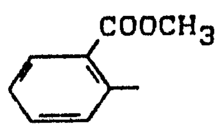 | 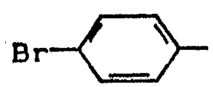 | $-OCH_3$ | 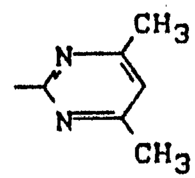 | H | 143 |
| 18 | 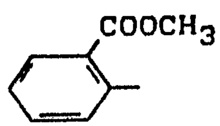 | 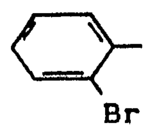 | $-OCH_3$ | 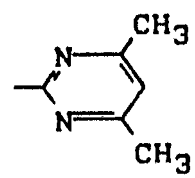 | H | 155 |
| 19 | 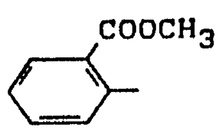 | 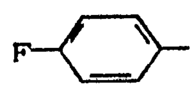 | $-OCH_3$ | 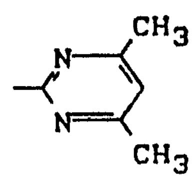 | H | 155 |

<u>Tabelle 3</u> - Fortsetzung

| Beisp.-Nr. | $R^1$ | $R^3$ | $R^4$ | 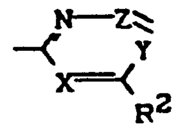 | M | Fp [°C] |
|---|---|---|---|---|---|---|
| 20 | 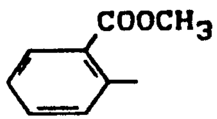 | 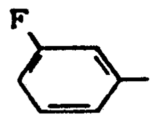 | $-OCH_3$ | 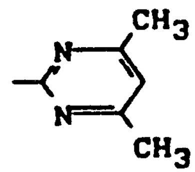 | H | 148 |
| 21 | 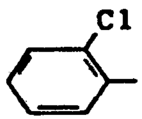 | 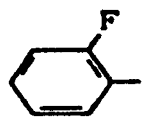 | $-OCH_3$ | 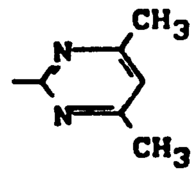 | H | 152 |
| 22 | 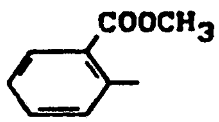 | 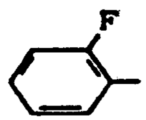 | $-OCH_3$ | 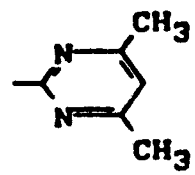 | H | 158 |
| 23 | 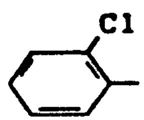 | 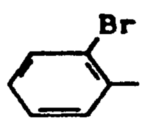 | $-OCH_3$ | 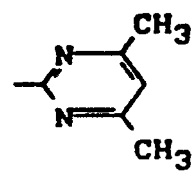 | H | 143 |
| 24 | 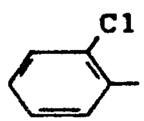 | 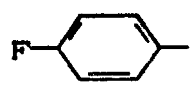 | $-OCH_3$ | 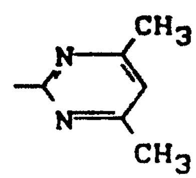 | H | 147 |

Tabelle 3 - Fortsetzung

| Beisp.-Nr. | $R^1$ | $R^3$ | $R^4$ | 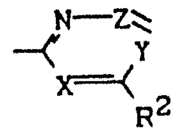 $R^2$ | M | Fp [°C] |
|---|---|---|---|---|---|---|
| 25 | 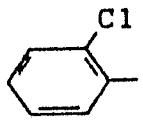 | 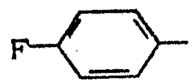 | -OH | 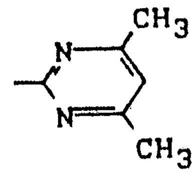 | H | 186 |
| 26 | 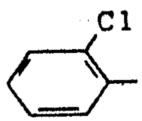 | 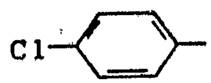 | -OH | 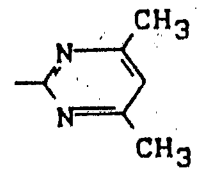 | H | 153 |
| 27 | 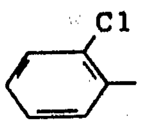 | 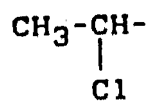 | $-OCH_3$ | 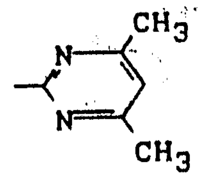 | H | (Öl) |
| 28 | 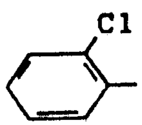 | $CCl_3$- | $-OCH_3$ | 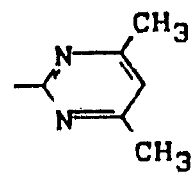 | H | 128 |
| 29 | 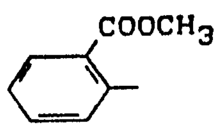 | $CCl_3$- | $-OCH_3$ | 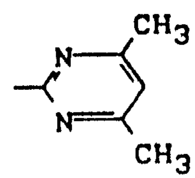 | H | 148 |

Tabelle 3 - Fortsetzung

| Beisp.-Nr. | $R^1$ | $R^3$ | $R^4$ | 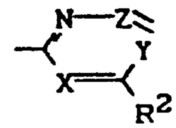 | M | Fp [°C] |
|---|---|---|---|---|---|---|
| 30 | 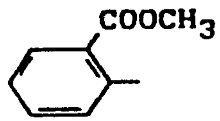 | 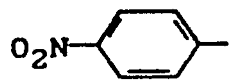 | $-OCH_3$ | 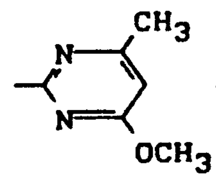 | 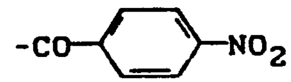 | |
| 31 | 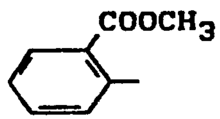 | 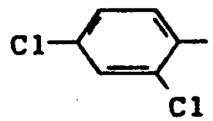 | $-OCH_3$ | 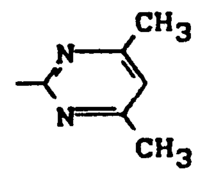 | 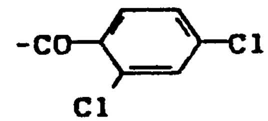 | 155 |
| 32 | 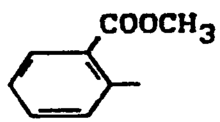 | 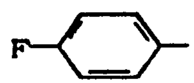 | $-OCH_3$ | 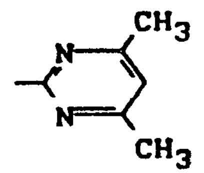 | 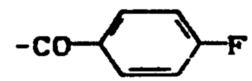 | 146 |
| 33 | 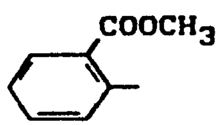 | 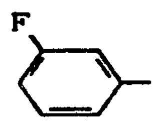 | $-OCH_3$ | 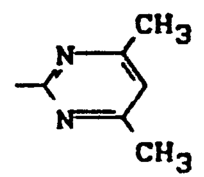 | 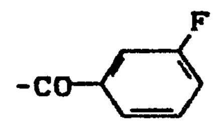 | 151 |
| 34 | 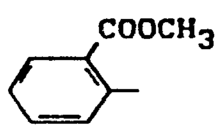 | 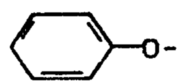 | $-OCH_3$ | 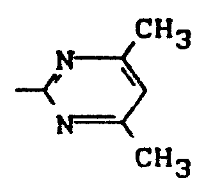 | H | 148 |

Tabelle 3 - Fortsetzung

| Beisp.-Nr. | $R^1$ | $R^3$ | $R^4$ | 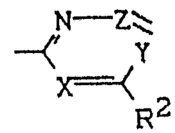 $R^2$ | M | Fp [°C] |
|---|---|---|---|---|---|---|
| 35 | 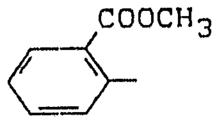 | $CH_3$-NH- | $-OCH_3$ | 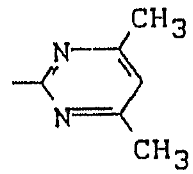 | H | 146 |
| 36 | 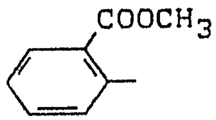 | 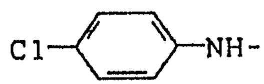 | $-OCH_3$ | 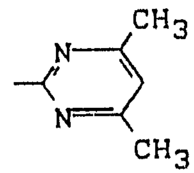 | H | 173 |
| 37 | 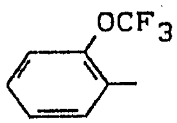 | 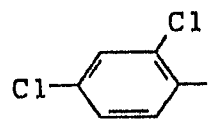 | $-OCH_3$ | 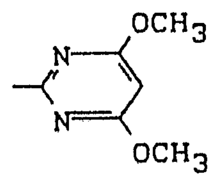 | H | 133 |
| 38 | 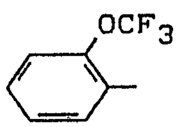 | $CH_3NH$- | $-OCH_3$ | 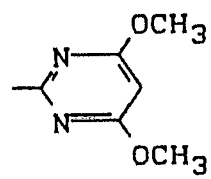 | H | |
| 39 | 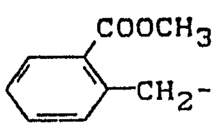 | $CH_3$ | $-OCH_3$ | 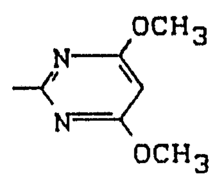 | H | |
| 40 | 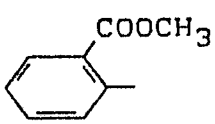 | 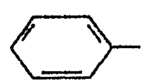 | $-N(CH_3)_2$ | 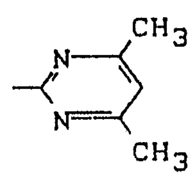 | H | 199 |

Herstellung von Ausgangsprodukten der Formel (V)

Beispiel (V-1)

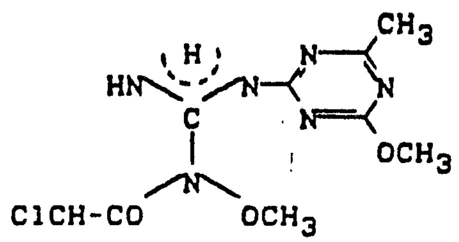

7,5 g (0,05 Mol) Dichloracetylchlorid werden unter Rühren zu einer auf 0 °C bis +5 °C gehaltenen Mischung aus 8,8 g (0,05 Mol) N'-(4-Methoxy-6-methyl-s-triazin-2-yl)-N''-methoxy-guanidin, 6,0 g (0,054 Mol) 1,4-Diazabicyclo-[2,2,2]-octan und 100 ml Methylenchlorid gegeben. Nach 15-stündigem Rühren bei 20 °C wird mit Wasser gewaschen, mit Natriumsulfat getrocknet, filftriert und unter vermindertem Druck eingeengt.

Man erhält 11,0 g (71 % der Theorie) N'-(4-Methoxy-6-methyl-s-triazin-2-yl)-N''-methoxy-N''-dichloracetyl-guanidin als hochviskoses Öl (von nicht bestimmbarem Brechungsindex).

Beispiel (V-2)

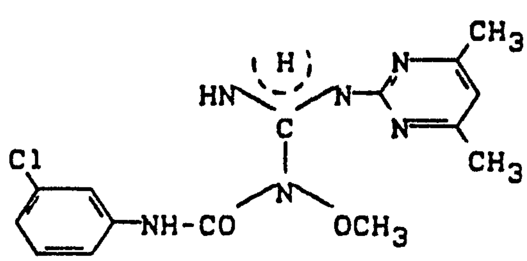

Eine Mischung aus 9,8 g (0,05 Mol) N'-(4,6-Dimethyl-pyrimidin-2-yl)-N''-methoxy-guanidin, 7,7 g (0,05 Mol) 3-Chlor-phenyl-isocyanat und 80 ml Dioxan wird eine Stunde bei 20 °C gerührt. Das kristallin angefallene Produkt wird durch Absaugen isoliert.

Man erhält 12,0 g (69 % der Theorie) N'-(4,6-Dimethyl-pyrimidin-2-yl)-N''-methoxy-N''-(3-chlor-phenylaminocarbonyl)-guanidin vom Schmelzpunkt 169 °C.

Analog Beispiel (V-1) und (V-2) können die folgenden Guanidin-Derivate der Formel (V) hergestellt werden:

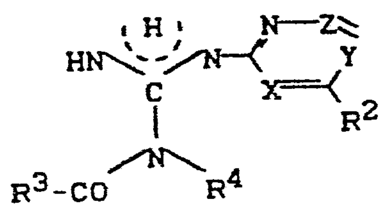

(V)

Tabelle 4

| Beisp.-Nr. | $R^3$ | $R^4$ | 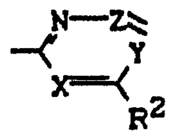 | Fp [°C] |
|---|---|---|---|---|
| (V-3) | $CH_3$ | $-OCH_3$ | 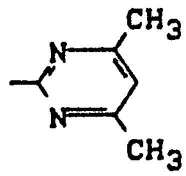 | 74 - 76 |
| (V-4) | 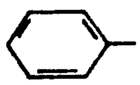 | $-OCH_3$ | 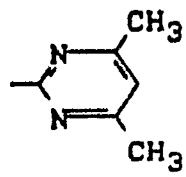 | 112 - 118 |
| (V-5) | $CH_3$-NH- | $-OCH_3$ | 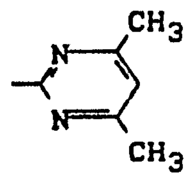 | 114 (Zers.) |
| (V-6) | $H_3C$ 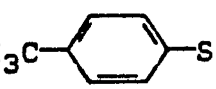 $SO_2$-NH- | $-OCH_3$ | 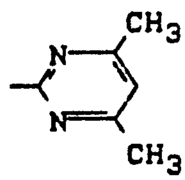 | 162 |
| (V-7) | $O_2N$ 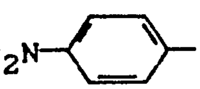 | $-OCH_3$ | 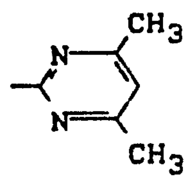 | 148 |
| (V-8) | Cl 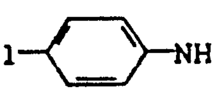 NH- | $-OCH_3$ | 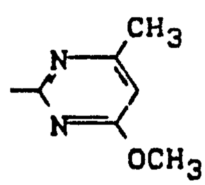 | |

Beispiel A

Pre-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z. B. die folgenden Verbindungen der Herstellungsbeispiele eine sehr gute Wirksamkeit: (16), (17), (18), (20) und (22)

Beispiel B

Post-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigen z. B. die folgenden Verbindungen der Herstellungsbeispiele eine sehr gute Wirksamkeit gegen Unkräuter: (12), (13) und (18).
Die Verbindung (12) zeigt außerdem eine ausgezeichnete Weizenverträglichkeit.

Patentansprüche

1) Acylierte Sulfonylguanidine der allgemeinen Formel (I)

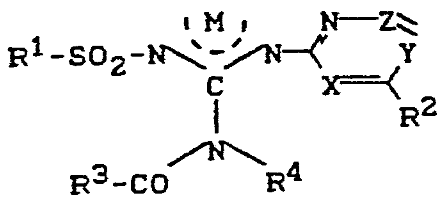

(I)

in welcher

$R^1$ für einen gegebenenfalls substituierten Rest aus der Reihe Alkyl, Aralkyl und Aryl mit jeweils bis zu 10 Kohlenstoffatomen oder für einen gegebenenfalls substituierten und/oder gegebenenfalls anellierten fünf- oder sechsgliedrigen aromatischen Heterocyclus, welcher ein Sauerstoff- oder ein Schwefelstoffatom und/oder 1 bis 2 Stickstoffatome enthält, steht,

$R^2$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine -$CR^5$-Gruppierung steht, worin

$R^5$ für Wasserstoff, Halogen, Cyano, $C_1$-$C_4$-Alkyl, Formyl, $C_1$-$C_4$-Alkyl-carbonyl oder $C_1$-$C_4$-Alkoxy-carbonyl steht,

Z für Stickstoff oder eine -$CR^6$-Gruppierung steht, worin

$R^6$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

in welcher weiter

$R^3$ für einen Rest $R^7$ oder für einen Rest -$NHR^8$ steht, wobei

$R^7$ für einen gegebenenfalls substituierten Rest aus der Reihe Alkyl, Alkenyl, Alkinyl, Alkoxy, Alkenyloxy, Alkinyloxy, Cycloalkyl, Dialkylamino, Aralkyl, Aryl, Aralkoxy, Aryloxy, Alkylthio, Aralkylthio und Arylthio mit jeweils bis zu 10 Kohlenstoffatomen steht und

$R^8$ für einen gegebenenfalls substituierten Rest aus der Reihe Alkyl, Cycloalkyl, Aralkyl, Aryl oder Arylsulfonyl mit jeweils bis zu 10 Kohlenstoffatomen steht,

in welcher weiter

$R^4$ für Wasserstoff oder für einen gegebenenfalls substituierten Rest aus der Reihe Alkyl, Alkenyl, Alkinyl, Cycloalkyl, Aralkyl und Aryl mit jeweils bis 10 zu Kohlenstoffatomen steht oder

$R^4$ für den Rest $-OR^9$ steht, worin

$R^9$ für Wasserstoff oder einen gegebenenfalls substituierten Rest aus der Reihe Alkyl, Alkenyl, Alkinyl, Cycloalkyl, Cycloalkylalkyl, Aralkyl und Aryl mit jeweils bis zu 10 Kohlenstoffatomen steht,

in welcher weiter

$R^4$ für den Rest $-NR^{10}R^{11}$ steht, worin

$R^{10}$ für Wasserstoff oder gegebenenfalls substituiertes $C_1$-$C_4$-Alkyl steht und

$R^{11}$ für Wasserstoff oder einen gegebenenfalls substituierten Rest aus der Reihe Alkyl, Alkenyl, Alkinyl, Cycloalkyl, Aralkyl, Aryl, Alkylcarbonyl, Alkoxycarbonyl, Alkylsulfonyl, Arylsulfonyl mit jeweils bis zu 10 Kohlenstoffatomen oder für einen gegebenenfalls substituierten fünf- oder sechsgliedriegen aromatischen Heterocyclus, welcher ein Sauerstoff- oder ein Schwefelatom und/oder 1 bis 3 Stickstoffatome enthält, steht und

M für Wasserstoff, ein Metalläquivalent oder für den Rest $-COR^7$ steht, worin

$R^7$ die oben angegebenen Bedeutungen hat,

sowie Addukte von Verbindungen der Formel (I) mit starken Säuren.

2) Verfahren zur Herstellung von acylierten Sulfonylguanidinen der allgemeinen Formel (I) gemäß Anspruch 1, dadurch gekennzeichnet, daß man

(a) für den Fall, daß $R^3$ für den Rest $R^7$ steht, worin $R^7$ die oben angegebenen Bedeutungen hat, und M für Wasserstoff steht, Sulfonylguanidin-Derivate der Formel (II)

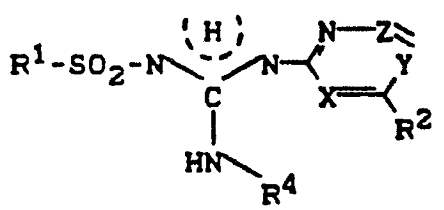

(II)

in welcher

X, Y, Z, $R^1$, $R^2$ und $R^4$ die oben angegebenen Bedeutungen haben,

mit Acylierungsmitteln der Formel (III)

$R^7COW$ (III)

in welcher

W für Halogen oder für den Rest $R^7COO-$ steht und

$R^7$ die oben angegebenen Bedeutungen hat,

gegebenenfalls in Gegenwart von Säureakzeptoren und gegebenenfalls in Gegenwart von Verdünnungsmitteln umsetzt;

oder daß man

(b) für den Fall, daß $R^3$ für den Rest $-NHR^8$ steht, worin $R^8$ die oben angegebenen Bedeutungen hat, und M für Wasserstoff steht, Sulfonylguanidin-Derivate der Formel (II)

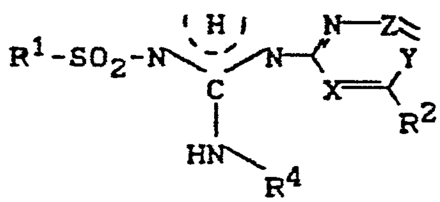

(II)

in welcher

X, Y, Z, $R^1$, $R^2$ und $R^4$ die oben angegebenen Bedeutungen haben,

mit Isocyanaten der Formel (IV)

$$R^8\text{-NCO} \qquad (IV)$$

in welcher

$R^8$ die oben angegebenen Bedeutungen hat,

gegebenenfalls in Gegenwart von Katalysatoren und gegebenenfalls in Gegenwart von Verdünnungsmitteln umsetzt;

oder daß man

(c) für den Fall, daß M für Wasserstoff steht, Guanidin-Derivate der Formel (V)

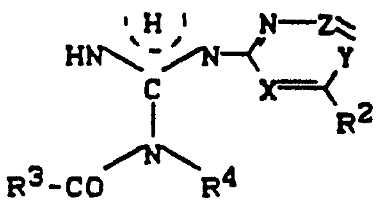

(V)

in welcher

X, Y, Z, $R^2$, $R^3$ und $R^4$ die oben angegebenen Bedeutungen haben,

mit Sulfonsäurechloriden der Formel (VI)

$$R^1\text{-}SO_2Cl \quad (VI)$$

in welcher

$R^1$ die oben angegebenen Bedeutungen hat,

in Gegenwart von Säureakzeptoren und gegebenenfalls in Gegenwart von Verdünnungsmitteln umsetzt;

oder daß man

(d) für den Fall, daß M für ein Metalläquivalent steht, die nach den oben unter (a), (b) und (c) angegebenen Verfahren erhältlichen Verbindungen der Formel (I), in welcher M für Wasserstoff steht und X, Y, Z, $R^1$, $R^2$, $R^3$ und $R^4$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder daß man

(e) für den Fall, daß 1:1-Addukte von Verbindungen der Formel (I) mit starken Säuren herzustellen sind,

Verbindungen der Formel (I), in welcher M für Wasserstoff steht und X, Y, Z, $R^1$, $R^2$, $R^3$ und $R^4$ die oben angegebenen Bedeutungen haben,

mit starken Säuren, gegebenenfalls in Gegenwart von Verdünnungsmitteln umsetzt;

oder daß man

(f) für den Fall, daß M für den Rest $R^7CO-$ steht, worin $R^7$ die oben angegebenen Bedeutungen hat,

(α) die nach den oben unter (a), (b) und (c) angegebenen Verfahren erhältlichen Verbindungen der Formel (I), in welcher M für Wasserstoff steht und X, Y, Z, $R^1$, $R^2$, $R^3$ und $R^4$ die oben angegebenen Bedeutungen haben,

mit Acylierungsmitteln der Formel (III)

$R^7COW$ (III)

in welcher

W und $R^7$ die oben angegebenen Bedeutungen haben,

gegebenenfalls in Gegenwart von Säureakzeptoren und gegebenenfalls in Gegenwart von Verdünnungsmitteln umsetzt,

oder daß man

(β) Sulfonylguanidin-Derivate der Formel (II)

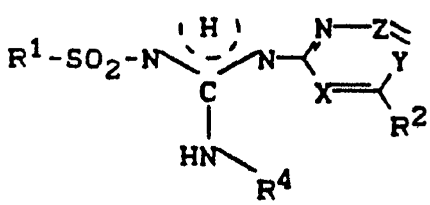

(II)

in welcher

X, Y, Z, $R^1$, $R^2$ und $R^4$ die oben angegebenen Bedeutungen haben,

mit mindestens der zweifachen molaren Menge Acylierungsmittel der Formel (III)

$R^7COW$ (III)

in welcher

W und $R^7$ die oben angegebenen Bedeutungen haben,

in Gegenwart von Säureakzeptoren und gegebenenfalls in Gegenwart von Verdünnungsmitteln umsetzt.

3) Herbizide Mittel, gekennzeichnet durch einen Gehalt an mindestens einem acylierten Sulfonylguanidin der allgemeinen Formel (I) gemäß Anspruch 1.

4) Verwendung von acylierten Sulfonylguanidinen der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man acylierte Sulfonylguanidine der allgemeinen Formel (I) gemäß Anspruch 1 mit Streckmitteln und/oder oberflächenaktiven Mitteln vermischt.

6) Guanidin-Derivate der allgemeinen Formel (V)

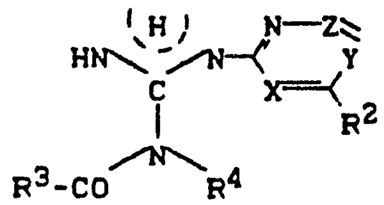

(V)

in welcher

$R^2$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine -$CR^5$-Gruppierung steht, worin

$R^5$ für Wasserstoff, Halogen, Cyano, $C_1$-$C_4$-Alkyl, Formyl, $C_1$-$C_4$-Alkyl-carbonyl oder $C_1$-$C_4$-Alkoxy-carbonyl steht,

Z für Stickstoff oder eine -$CR^6$-Gruppierung steht, worin

$R^6$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

in welcher weiter

$R^3$ für einen Rest $R^7$ oder für einen Rest $-NHR^8$ steht, worin

$R^7$ für einen gegebenenfalls substituierten Rest aus der Reihe Alkyl, Alkenyl, Alkinyl, Alkoxy, Alkenyloxy, Alkinyloxy, Cycloalkyl, Dialkylamino, Aralkyl, Aryl, Aralkoxy, Aryloxy, Alklylthio, Aralkylthio und Arylthio mit jeweils bis zu 10 Kohlenstoffatomen steht und

$R^8$ für einen gegebenenfalls substituierten Rest aus der Reihe Alkyl, Cycloalkyl, Aralkyl, Aryl oder Arylsulfonyl mit jeweils bis zu 10 Kohlenstoffatomen steht,

in welcher weiter

$R^4$ für Wasserstoff oder für einen gegebenenfalls substituierten Rest aus der Reihe Alkyl, Alkenyl, Alkinyl, Cycloalkyl, Aralkyl und Aryl mit jeweils bis zu 10 Kohlenstoffatomen steht oder

$R^4$ für den Rest $-OR^9$ steht, worin

$R^9$ für Wasserstoff oder einen gegebenenfalls substituierten Rest aus der Reihe Alkyl, Alkenyl, Alkinyl, Cycloalkyl, Cycloalkylalkyl, Aralkyl und Aryl mit jeweils bis zu 10 Kohlenstoffatomen steht,

in welcher weiter

$R^4$ für den Rest $-NR^{10}R^{11}$ steht, worin

$R^{10}$ für Wasserstoff oder gegebenenfalls substituiertes $C_1$-$C_4$-Alkyl steht und

$R^{11}$ für Wasserstoff oder einen gegebenenfalls substituierten Rest aus der Reihe Alkyl, Alkenyl, Alkinyl, Cycloalkyl, Aralkyl, Aryl, Alkylcarbonyl, Alkoxycarbonyl, Alkylsulfonyl, Arylsulfonyl mit jeweils bis zu 10 Kohlenstoffatomen oder für einen gegebenenfalls substituierten fünf- oder sechsgliedriegen aromatischen Heterocyclus, welcher ein Sauerstoff- oder ein Schwefelatom und/oder 1 bis 3 Stickstoffatome enthält, steht.

7) Verfahren zur Herstellung von Guanidin-Derivaten der allgemeinen Formel (V) gemäß Anspruch 6, dadurch gekennzeichnet, daß man Guanidine der allgemeinen Formel (VII)

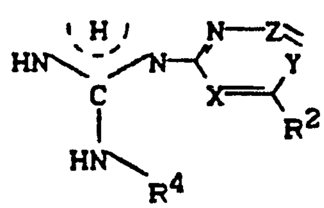

(VII)

in welcher

X, Y, Z, $R^2$ und $R^4$ die in Anspruch 6 angegebenen Bedeutungen haben,

(a) für den Fall, daß $R^3$ für den Rest $R^7$ steht, worin $R^7$ die in Anspruch 6 angegebenen Bedeutungen hat, mit Acylierungsmitteln der allgemeinen Formel (III)

$$R^7COW \qquad (III)$$

in welcher

$R^7$ und W die in Anspruch 2 angegebenen Bedeutungen haben,

gegebenenfalls in Gegenwart von Säureakzeptoren und gegebenenfalls in Gegenwart von Verdünnungsmitteln umsetzt,

oder

(b) für den Fall, daß $R^3$ für den Rest $-NHR^8$ steht, worin $R^8$ die in Anspruch 6 angegebenen Bedeutungen hat, mit Isocyanaten der allgemeinen Formel (IV)

$$R^8\text{-NCO} \qquad (IV)$$

in welcher

$R^8$ die in Anspruch 2 angegebenen Bedeutungen hat,

gegebenenfalls in Gegenwart von Katalysatoren und gegebenenfalls in Gegenwart von Verdünnungsmitteln umsetzt.